# EUROPEAN PATENT SPECIFICATION

(11) **EP 3 544 637 B1**
(45) Date of publication and mention of the grant of the patent: **11.11.2020**
(21) Application number: 17816489.3
(22) Date of filing: 23.11.2017
(51) Int. Cl.: A61K 47/69, A61K 47/64, A61P 31/04, A61K 39/095, A61K 39/015, A61K 39/112, A61P 33/06

(54) **NATIVE OMV-ANTIGEN CONJUGATES AND USE THEREOF**
NATIVE OMV-ANTIGEN-KONJUGATE UND DEREN VERWENDUNGEN
CONJUGUÉS ANTIGÈNES-VME NATIFS ET LEUR UTILISATION

(30) Priority: 25.11.2016 GB 201619949; 27.07.2017 GB 201712101
(43) Date of publication of application: 02.10.2019
(73) Proprietor: GlaxoSmithKline Biologicals S.A., 1330 Rixensart (BE)
(72) Inventor: DI BENEDETTO, Roberta, 53100 Siena (IT); MICOLI, Francesca, 53100 Siena (IT); SAUL, Allan James, 53100 Siena (IT)
(74) Representative: Sanderson, Andrew John
(86) International application number: PCT/EP2017/080151
(87) International publication number: WO 2018/096013

(56) References cited:
- EP-A1- 0 467 714
- WO-A1-2016/083583
- WO-A1-2016/184860
- SEYED DAVAR SIADAT ET AL: "Preparation and Evaluation of a New Lipopolysaccharide-based Conjugate as a Vaccine Candidate for Brucellosis", OSONG PUBLIC HEALTH AND RESEARCH PERSPECTIVES,, vol. 6, no. 1, 1 February 2015 (2015-02-01), pages 9-13, XP009503435, ISSN: 2210-9099, DOI: 10.1016/J.PHRP.2014.10.012 [retrieved on 2014-12-18]
- GERRITZEN MATTHIAS J H ET AL: "Bioengineering bacterial outer membrane vesicles as vaccine platform", BIOTECHNOLOGY ADVANCES, ELSEVIER PUBLISHING, BARKING, GB, vol. 35, no. 5, 15 May 2017 (2017-05-15), pages 565-574, XP085118072, ISSN: 0734-9750, DOI: 10.1016/J.BIOTECHADV.2017.05.003
- ROLANDO PAJON ET AL: "Mutant Native Outer Membrane Vesicles Combined with a Serogroup A Polysaccharide Conjugate Vaccine for Prevention of Meningococcal Epidemics in Africa", PLOS ONE, vol. 8, no. 6, 21 June 2013 (2013-06-21), page e66536, XP055163430, DOI: 10.1371/journal.pone.0066536
- DEVI S J N ET AL: "Binding diversity of monoclonal antibodies to alpha(2-->8) polysialic acid conjugated to outer membrane vesicle via adipic acid dihydrazide", FEMS IMMUNOLOGY AND MEDICAL MICROBIOLOGY, ELSEVIER SCIENCE B.V., AMSTERDAM, NL, vol. 14, no. 4, 1 July 1996 (1996-07-01), pages 211-220, XP002315243, ISSN: 0928-8244
- DEVI S J N ET AL: "PRECLINICAL EVALUATION OF GROUP B NEISSERIA MENINGITIDIS AND ESCHERICHIA COLI K92 CAPSULAR POLYSACCHARIDE-PROTEIN CONJUGATE VACCINES IN JUVENILE RHESUS MONKEYS", INFECTION AND IMMUNITY,, vol. 65, no. 3, 1 March 1997 (1997-03-01), pages 1045-1052, XP000872520, ISSN: 0019-9567
- FUKASAWA L O ET AL: "Neisseria meningitidis serogroup C polysaccharide and serogroup B outer membrane vesicle conjugate as a bivalent meningococcus vaccine candidate", VACCINE, ELSEVIER, AMSTERDAM, NL, vol. 17, no. 23-24, 6 August 1999 (1999-08-06), pages 2951-2958, XP004173605, ISSN: 0264-410X, DOI: 10.1016/S0264-410X(99)00177-2
- SHARIFAT SALMANI ET AL., 2008: J., 2008,
- FRASCH ET AL., 1978: JOURNAL OF EXPERIMENTAL MEDICINE, 1978,
- ZOLLINGER ET AL., 1997: INFECTION AND IMMUNITY, 1997,
- SARVAMANGALA J.N. DEVI ET AL., 1991: PNAS, 1991,

## Description

### TECHNICAL FIELD

This invention is in the field of conjugating non-detergent extracted, native outer membrane vesicles (nOMVs) to antigens, via a divalent linker, to form nOMV-antigen conjugates. The conjugates are useful for immunisation.

### BACKGROUND ART

Conjugation of antigens to carriers is an established procedure for improving immunogenicity, particularly for polysaccharides. For instance, bacterial capsular polysaccharides (CPS) are naturally T-cell independent antigens which give rise to an immune response that lacks several important properties. Conjugation to a carrier converts these saccharides to T-cell dependent antigens which can then produce an immunological memory effect, and also elicit protective immune responses in young children.

One known carrier in such conjugates is the 'OMPC' outer membrane protein complex, formed from *N.meningitidis* vesicles (*e.g.* see EP-0467714), which has been included as the carrier in approved *H.influenzae* B conjugate vaccines. OMPC has also been used as the carrier in protein conjugates. For example, Wu et al. (PNAS USA 2006; 103(48): 18243-18248) report that conjugation of Pfs25H to OMPC resulted in a Pfs25H-OMPC conjugate vaccine that was >1,000 times more potent in generating anti-Pfs25H ELISA reactivity in mice than a similar dose of Pfs25H alone.

Conjugation of OMPC to Pfs25H protein can be achieved by reacting maleimide-activated Pfs25H with thiolated outer membrane proteins within OMPC (see *e.g.* WO2006/124712), as shown in Scheme 1.

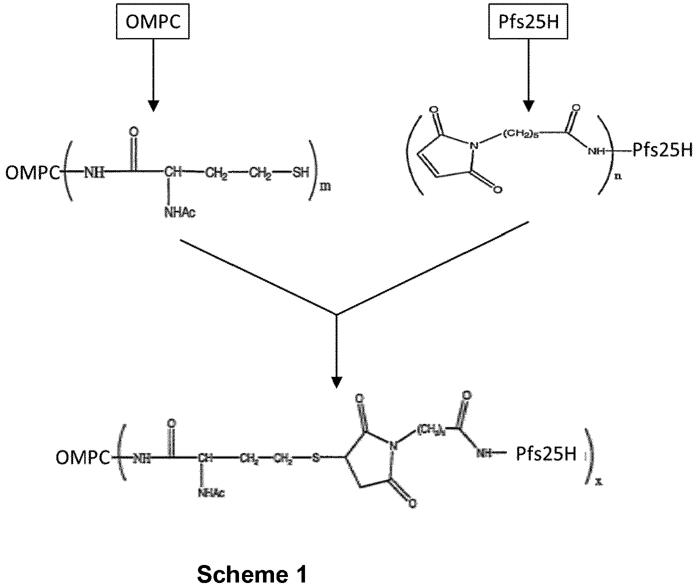

As shown in the above Scheme 1 and according to general procedures of the prior art, the conjugation methods and conjugates thereof contemplate the use of a divalent heterobifunctional linker, *i.e.* a linker moiety having the terminal ends bearing different functional groups. This is mainly to avoid cross linking of the vesicle-linker intermediate with another vesicle particle rather than with the selected antigen. In practice, according to the prior art, the different ends of the linker are selected depending on the reactive groups on the vesicle and the selected antigen involved in the process, in order to have a selective reaction with the intended part, namely the vesicle on one side and the antigen on the other end side. These methodologies, however, suffer of some drawbacks, mainly related to the selection and functionalization of the linkers, thus posing some limitation on the choice of the vesicle and antigen to be coupled together. The Applicant has now found that when native, non-detergent extracted Outer Membrane Vesicles (nOMVs) are used as starting vesicles it is possible to use a divalent linker suitable for the connection with a nOMV surface protein on one side and with a selected antigen on the other end, thus providing a final conjugate that still presents the immunogenic activity of both the nOMV and the antigen. Surprisingly, even when the linker used in the present invention shows identical terminal functional groups, the conjugation of the nOMV with the selected antigen is achieved substantially without the formation of vesicle aggregates or side products, detrimental for the conjugation reaction.

### SUMMARY OF THE INVENTION

The present invention is defined by the claims. In a first aspect, the invention refers to an immunogenic nOMV-linker-antigen conjugate comprising a native Outer Membrane Vesicle (nOMV) obtained by a detergent free process, having at least a surface protein residue connected to at least a selected foreign antigen by a divalent homobifunctional linker.

In a further aspect, the invention refers to a process for preparing said conjugate, comprising the steps of:
i) reacting at least a nOMV surface protein residue with the first terminal portion of a divalent linker to obtain a nOMV-linker intermediate, and
ii) reacting said nOMV-linker intermediate with at least one selected foreign antigen via the second terminal portion of the divalent linker, thus obtaining the nOMV-linker-antigen conjugate of the invention.

The linker is a divalent homobifunctional linker, *i.e.* having the same terminal functionalities.

In a further aspect, the invention refers to the nOMV-linker intermediate obtained (or obtainable) by the above indicated step i), and its use for the preparation of nOMV-linker-antigen conjugate of the invention.

In one additional aspect, the invention also refers to the above nOMV-linker-antigen conjugate for use as an immunogenic compound, particularly for the preparation of an immunogenic composition or vaccine.

In a further aspect, the immunogenic conjugates according to the invention comprise a nOMV having at least a surface protein moiety connected to a first antigen by a divalent linker, wherein said first antigen is further connected to a second different antigen.

In a still further aspect, the immunogenic conjugates of the invention comprises a nOMV having at least a surface protein moiety connected to a first antigen by a divalent linker, and at least another surface protein moiety connected to a second different antigen by a divalent linker, wherein the divalent linker connecting the first antigen and the divalent linker connecting the second agent are independently the same or different to each other.

In a still further aspect, the invention refers to an immunogenic composition or a vaccine, comprising the above indicated conjugate and at least one pharmaceutically acceptable carrier or adjuvant; and to the composition for use in a method for raising an immune response in a vertebrate, comprising the administration of said composition or vaccine.

In a further aspect, the invention also refers to the use of nOMV for the preparation of immunogenic polyvalent nOMV-linker-antigen conjugates.

### BRIEF DESCRIPTION OF THE DRAWINGS

Figure 1 shows anti-CSP (1A) and anti-OAg (1B) IgG titers after immunisation with CSP or its fragment (NANP)₃ conjugated to *S.* Typhimurium nOMV by SH-malemido chemistry, compared to CSP physically mixed with said nOMV (formulated with no adjuvant). CD1 female mice, 5 weeks old (8 per group) were subcutaneously immunised at days 0 and 28 with 2 µg CSP/(NANP)₃ per dose. Titres were measured at days 0, 14, 28 and 42.
Figure 2: Anti-Pfs25 IgG response induced in mice (200 µL per dose SC injected at days 0 and 28, bleeds at days 0, 14, 27 and 42) by nOMV conjugates of the invention produced by conjugating S. Typhimurium nOMV with Pfs25 antigen, via BS3 linker.
Figure 3: Anti-fHbp IgG response induced in mice (200 µL per dose SC injected at days 0 and 28, bleeds at days 0, 14, 27 and 42) by nOMV conjugates of the invention produced by conjugating *S.* Typhimurium nOMV with fHbp antigen, via BS3 linker.
Figure 4: Anti-MenA IgG response induced in mice by nOMV conjugates of the invention produced by conjugating MenB nOMV with MenA antigen, via SIDEA linker, compared with MenA-MenC bivalent MenBGMMA conjugate, GMMA, CRM conjugate and mixture of MenA and GMMA (not conjugated).
Figure 5: Anti-MenC IgG response induced in mice by nOMV conjugates of the invention produced by conjugating MenB nOMV with MenC antigen, via SIDEA linker, compared with MenA-MenC bivalent MenBGMMA conjugate, GMMA, CRM conjugate and mixture of MenC and GMMA (not conjugated).

### DETAILED DESCRIPTION OF THE INVENTION

To facilitate an understanding of the present invention, a number of terms and phrases are defined below.

As used in the present disclosure and claims, the singular forms "a," "an," and "the" include plural forms unless the context clearly dictates otherwise; *i.e.,* "a" means "one or more" unless indicated otherwise.

The terms "about" or "approximately" mean roughly, around, or in the regions of. The terms "about" or "approximately" further mean within an acceptable contextual error range for the particular value as determined by one of ordinary skill in the art, which will depend in part on how the value is measured or determined, *i.e.* the limitations of the measurement system or the degree of precision required for a particular purpose, *e.g.* the amount of a nutrient within a feeding formulation. When the terms "about" or "approximately" are used in conjunction with a numerical range, it modifies that range by extending the boundaries above and below the numerical values set forth. For example "between about 0.2 and 5.0 mg/ml" means the boundaries of the numerical range extend below 0.2 and above 5.0 so that the particular value in question achieves the same functional result as within the range. For example, "about" and "approximately" can mean within 1 or more than 1 standard deviation as per the practice in the art. Alternatively, "about" and "approximately" can mean a range of up to 20%, preferably up to 10%, more preferably up to 5%, and more preferably up to 1% of a given value.

The term "and/or" as used in a phrase such as "A and/or B" is intended to include "A and B," "A or B," "A," and "B." Likewise, the term "and/or" as used in a phrase such as "A, B, and/or C" is intended to encompass each of the following embodiments: A, B, and C; A, B, or C; A or C; A or B; B or C; A and C; A and B; B and C; A (alone); B (alone); and C (alone).

Unless specified otherwise, all of the designations "A%-B%," "A-B%," "A% to B%," "A to B%," "A%-B," "A% to B" are given their ordinary and customary meaning. In some embodiments, these designations are synonyms.

The terms "substantially" or "substantial" mean that the condition described or claimed functions in all important aspects as the standard described. Thus, "substantially free" is meant to encompass conditions that function in all important aspects as free conditions, even if the numerical values indicate the presence of some impurities or substances. "Substantial" generally means a value greater than 90%, preferably greater than 95%, most preferably greater than 99%. Where particular values are used in the specification and in the claims, unless otherwise stated, the term "substantially" means with an acceptable error range for the particular value.

An "effective amount" means an amount sufficient to cause the referenced effect or outcome. An "effective amount" can be determined empirically and in a routine manner using known techniques in relation to the stated purpose.

As used herein, "heterologous" means the two or more referenced molecules or structures are derived from a different organism. For example, for a nOMV, a heterologous antigen is one that is derived from a different organism than the nOMV vesicle to which it is appended. "Homologous" as used herein means the two or more referenced molecules or structures are derived from the same organism.

As used herein, "foreign" means the two or more referenced molecules or structures are not naturally associated with each other. For example, a selected antigen that is herein intended to be "foreign to" a nOMV surface saccharide herein means the antigen is not naturally or innately conjugated to the surface saccharide and is, therefore, not naturally conjugated to the nOMV molecule even though the antigen and the saccharide (or nOMV molecule) may originate from the same organism. In this way, a foreign antigen is not necessarily a heterologous antigen but a heterologous antigen is a foreign antigen.

"Sequence identity" can be determined by the Smith-Waterman homology search algorithm as implemented in the MPSRCH program (Oxford Molecular), using an affine gap search with parameters gap open penalty=12 and gap extension penalty=1, but is preferably determined by the Needleman-Wunsch global alignment algorithm (see *e.g.* Rubin (2000) Pediatric. Clin. North Am. 47:269-285), using default parameters (*e.g.* with Gap opening penalty = 10.0, and with Gap extension penalty = 0.5, using the EBLOSUM62 scoring matrix). This algorithm is conveniently implemented in the needle tool in the EMBOSS package. Where the application refers to sequence identity to a particular SEQ ID, the identity is intended to be calculated over the entire length of that SEQ ID.

The term "% w/w" indicates the weight percentage of a given compound, over a different compound or over the whole content of a composition, as indicated.

Analogously, the term "% v/v" indicates the volume percentage of a given compound, over a different compound or over the whole content of a composition, as indicated.

As herein used, the term "saccharide (or sugar) moiety" comprises in its meaning mono saccharides, as well as polysaccharide units. It will be appreciated that saccharide moieties can exist in open and closed (ring) form and that, while closed forms are shown in structural formulae herein, open forms are also encompassed by the invention. Similarly, it will be appreciated that saccharide moieties can exist in pyranose and furanose forms and that, while pyranose forms are shown in structural formulae herein, furanose forms are also encompassed. Different anomeric forms of saccharide moieties are also encompassed.

The term "oligosaccharide" comprises in its meaning polysaccharides having from 3 to 10 monosaccharide units, as generally known in the art (see *e.g.* https://en.wikipedia.org/wiki/Oligosaccharide).

Unless otherwise provided, the term "polypeptide" refers to polypeptides of any length capable to act as a selected antigen. The amino acid polymer forming the polypeptide of the invention, may be linear or branched, it may comprise modified amino acids, and it may be interrupted by non-amino acids. The term also encompasses an amino acid polymer that has been modified naturally or by intervention; for example, disulfide bond formation, glycosylation, lipidation, acetylation, phosphorylation, or any other manipulation or modification, such as conjugation with a labelling component. Also included within the definition are, for example, polypeptides containing one or more analogues of an amino acid (including, for example, unnatural amino acids, etc.), as well as other modifications known in the art. Polypeptides can occur as single chains or associated chains.

"Average molecular weight" is intended to indicate the average molecular weight obtained by the ordinary arithmetic mean or average of the molecular masses of the individual component, e.g. amino acids in case of polypeptide conjugates.

The term "-OAg" (O-antigen) is used within the present invention to indicate an antigen functionality present in the lipopolysaccharides (LPS) or lipooligosaccharides (LOS) on the surface of the considered nOMV. In more details, the LPS are generally formed by three different portions, known as: lipidA (responsible for the toxicity of LPS), core oligosaccharide and the -OAg chain, a repetitive glycan polymer and major contributor to the serological specificity of bacteria.

The term "capsular polysaccharides/saccharides" (CPSs) indicates those saccharides which can be found in the layer that lies outside the cell envelope of bacteria, thus being part of the outer envelope of the bacterial cell itself. CPSs are expressed on the outermost surface of a wide range of bacteria, and in some cases even in fungi.

The term "suspension" comprises in its meaning a liquid medium presenting some precipitates or particulates or flocculating particles, differently from a solution where the medium is substantially free of any solid particles.

Unless otherwise provided, the term "conjugation" indicates the connection or linkage of the subjected entities, particularly referred to the nOMV and the selected antigen moieties, via a divalent linker.

By "immunologically effective amount", it is meant that the administration of that amount to an individual, either in a single dose or as part of a series, is effective for treatment or prevention. This amount can vary depending upon the health and physical condition of the individual to be treated, age, the taxonomic group of individual to be treated (*e.g.* non-human primate, primate, etc.), the capacity of the individual's immune system to synthesise antibodies, the degree of protection desired, the formulation of the vaccine, the treating doctor's assessment of the medical situation, and other relevant factors. It is expected that the amount will fall in a relatively broad range that can be determined through routine trials.

The term "nOMVs" herein indicates vesicles isolated from the medium and/or sheared from cells, and they are intact membrane vesicles not exposed to detergents or denaturating agents, *i.e.* non detergent extracted. The nOMVs of the invention present the outer membrane proteins (OMP) and/or lipopolysaccharide (LPS) in their native conformation and correct orientation in the natural membrane environment, and usually lack the cytoplasmatic components.

On the contrary, the term "OMV" or "dOMV" encompasses a variety of proteoliposomic vesicles obtained by disruption of the outer membrane of a Gram-negative bacterium typically by a detergent extraction process to form vesicles therefrom. Outer membrane protein complexes (*e.g.* OMPC from Neisseria meningitides) may be considered in such definition, since having three dimensional structure and composition similar to dOMV, and being isolated via detergent extraction procedures (see *e.g.* EP0467714, US4271147, US4459286 and US4830852). The detergent extraction process removes LPS and phospholipids, together with immunoprotective lipoproteins. Such removal changes the native vesicle structure and promotes aggregation. The aggregation may lead to consequent issues in terms of process development (yield, consistency of production and stability). Differently from nOMVs, characterized by defined homogeneous size distribution (typically in the range 20-250 nm, measured by Dynamic Light Scattering DLS technique), the dOMVs have an undefined heterogeneous size distribution (usually in the range 550-5500 nm as measured by Dynamic Light Scattering DLS technique) caused by detergent-induced vesicle aggregation (see for a general reference, Vaccine 28, 2010, 4810). The detergent extraction process also causes contamination of OMV containing composition (*e.g.* vaccines) with cytoplasmic proteins as a result of bacterial cell lysis.

According to prior art methodologies, dOMVs and nOMVs may be analysed and described in terms of size, shape and overall appearance of impurities or contaminating non-OMV materials (like vesicle aggregates or detergent residues in case of dOMVs) using the Transmission Electron Microscopy (TEM). For detailed references regarding the differences between dOMVs and nOMVs see *e.g.* van de Waterbeemd et al. J. Prot. Res. 12(4) (2013) 1898-1908 "Quantitative Proteomics Reveals Distinct Differences in the Protein Content of Outer Membrane Vesicle Vaccines"; and J. Klimentova et al. Microbiological Research 170 (2015) 1-9 "Methods of isolation and purification of the outer membrane vesicles from gram-negative bacteria".

The term "divalent homobifunctional linker" or "homologous linker" indicates a linking unit presenting two terminal ends bearing the same functional group, and able to react with the nOMV protein on one side, and with the selected antigen on the other side, where nOMV protein and selected antigen are as herein below described in details.

Similarly, the term "divalent heterobifunctional linker" or "heterologous linker" indicates a linking unit presenting two terminal ends bearing different functional groups, and able to specifically react with the nOMV protein on one side, and with the selected antigen on the other side, where nOMV protein and selected antigen are as herein below described in detail.

The term "divalent C₁-C₁₀ alkyl or alkenyl group" comprises in its meaning a divalent satured or unsatured alky or alkenyl group having 1 to 10 carbon atoms, such as methylene, ethylene, vinyl, allyl and the like.

As above introduced, the invention refers to nOMV-linker-antigen conjugates obtained by covalently connecting at least a protein unit on the nOMV surface to one or more selected foreign antigen(s), via suitable divalent linker(s). In other terms, and according to one aspect, the invention refers to nOMV-linker-antigen conjugates obtained (or obtainable) by the process of the invention as herein below described in more details.

Of note, the present conjugates are endowed with a remarkable immunogenic activity, as further supported by the herein experimental part. In fact, as well as being capable of inducing an immune response against the conjugated antigen, the conjugates of the invention are also capable of inducing an immune response against the nOMV component, differently from dOMV-antigen conjugates of the art where the immune activity relies mainly on the antigen portion and not on the detergent extracted vesicle. On the contrary, according to the present invention, the conjugation of the selected antigen to nOMV protein component, does not significantly impact on the ability of the nOMV to induce its own immune response. Of note, when nOMV and the selected antigen(s) are from different sources, the conjugates of the invention may be useful for instance for the preparation of multi-valent immunogenic compositions or vaccines based on both the nOMV and the conjugated antigen(s) activity.

Even further, the present invention surprisingly shows that a homobifunctional linker can be used in the preparation of nOMV-linker-antigen conjugates, without incurring in the prior art problems related *e.g.* to vesicle cross reaction or aggregation. In practice, the starting nOMV is functionalized with the homobifunctional linker by reacting proper functional groups of at least one vesicle surface protein with one end of the linker. By that, a nOMV-linker intermediate is covalently formed, still having the other end of the linker available for the subsequent reaction with the selected antigen. Thus, the second end of the linker will react with the selected antigen, in a specific and selective way, leading to the final nOMV-linker-antigen conjugate, and substantially avoiding intermediate cross reactions or aggregations. As indicated in the Example 8a, the same reaction when carried out considering a dOMV as starting vesicle leads to the formation of vesicle-linker-vesicle aggregates, which are not suitable for a subsequent reaction with the selected antigen. Surprisingly it has now found that not only the use of nOMV as starting vesicle can overcome the aggregation problems of the prior art, but also it is now possible to use a variety of bifunctional linkers that lead to the preparation of a series of nOMV-linker-antigens conjugates according to the invention, retaining the immunogenic profile of the conjugated components.

The conjugates of the invention offer several advantages compared to unconjugated antigens, for example the ability to act as a bivalent or multivalent immunogenic agent or vaccine as discussed above, and improved immunogenicity over unconjugated antigens (see Examples 5 and 7). In addition, conjugates of the invention have several advantages over the vesicle-protein conjugates that have been used to date. Firstly, the nOMVs can be prepared with fewer steps compared to dOMV conjugates in which antigens were coupled to dOMV proteins, and in particular without requiring the expensive and time consuming step of protein derivatisation. Secondly, the production of nOMVs can be more reliable and convenient than the preparation of dOMVs by detergent extraction. Even further, unreacted antigen from the conjugation mixture can be recycled for use in a further conjugation step, improving the efficiency of production of the conjugates as exemplified in Example 3.

In a first aspect, the invention refers to a conjugate comprising one or more different selected foreign antigen(s), each connected to a protein residue of a non-detergent extracted native Outer Membrane Vesicle (nOMV), through a divalent homobifunctional linker. Of note, the nOMV can be collected and isolated substantially without the use of detergents, differently for instance from dOMVs of the prior art obtained via a deoxycholate extraction or using zwitterionic detergents like Empigen BB (see *e.g.* US4707543) or similar.

In more details, the nOMVs are naturally-occurring membrane vesicles that form spontaneously during bacterial growth and are released into culture medium. They can be obtained *e.g.* by culturing bacteria in broth culture medium, separating whole cells from the smaller nOMVs in the broth culture medium (*e.g.* by filtration or by low-speed centrifugation to pellet only the cells and not the smaller vesicles), and then collecting the nOMVs from the cell-depleted medium (*e.g.* by filtration, by differential precipitation or aggregation, by high-speed centrifugation to pellet the vesicles). Strains for use in production of nOMVs can generally be selected on the basis of the amount of nOMVs produced in culture. As above set forth, the present nOMVs are characterised by the fact of being collected and isolated following a detergent-free procedure. Preferably, the nOMV are released into the fermentation broth and are purified using a centrifugation and subsequent filtration step (for a general reference see *e.g.* Clin. Vaccine Immunol. 2016 Apr; 23(4): 304-314). Still preferably, the nOMV are released into the fermentation broth and are purified using the following two consecutive Tangential Flow Filtration (TFF) steps: (i) a microfiltration in which the culture supernatant containing the nOMV is separated from the bacteria, and (ii) an ultrafiltration in which the nOMV are separated from soluble proteins (for a general reference see *e.g.* PLoS One. 2015; 10(8): e0134478). The thus obtained nOMV can then directly be used within the present invention without additional purification/isolation steps. The presently considered nOMVs have a preferred size distribution comprised from 20 to 250 nm, measured by Dynamic Light Scattering technique.

According to some embodiments, the nOMVs are prepared from wild-type bacteria or from bacteria which have been genetically manipulated generally to increase immunogenicity (*e.g.* to hyper-express immunogens), to reduce toxicity, to inhibit capsular saccharide synthesis, to down-regulate immunodominant antigen expression, and the like. They also may be prepared from hyperblebbing strains. The nOMVs of the invention may also express exogenous proteins on their surface and they may be endotoxin-depleted.

Preferably, the nOMVs to be used in the present invention are produced from genetically-modified bacterial strains that are mutated to enhance vesicles production, and optionally also to remove or modify antigens (*e.g.* lipid A) and/or to over-express homologous antigens or antigens from other organisms. Said preferred nOMV are also known as Generalized Modules of Membrane Antigens (GMMA) as *e.g.* described in PLoS One. 2015; 10(8): e0134478. Enhanced spontaneous generation of vesicles can be achieved, for example, by targeted deletion of proteins involved in maintenance of the membrane integrity. It has been observed that the outer surface of nOMVs substantially corresponds to the outer surface of the bacterium from which they are derived, preserving the membrane antigens (including *e.g.* lipopolysaccharides, lipooligosaccharides and lipoproteins) in the context of the membrane. Advantageously, the nOMVs used in the invention (unlike detergent-extracted dOMVs) retain these outer membrane components in their native conformation and correct orientation, better preserving immunogenicity against the bacterial strain from which they are derived.

Generally, the nOMVs for use in the present invention may be prepared from any suitable bacterium, where preferred bacteria include, *Neisseria* (*e.g.* in particular *N. meningitidis* of any serogroup including A, B, C, Y, X or W135, or from a non-pathogenic *Neisseria*), *Shigella* (such as *S*. *sonnei, S. flexneri, dysenteriae or boydii*), *Salmonella enterica serovars* (such as *Salmonella* Paratyphi, *Salmonella* Enteritidis, *Salmonella* Typhi or *Salmonella* Typhimurium), *Haemophilus influenzae* (*e.g.* non-typable *H. influenzae*), *Vibrio cholerae, Bordetella pertussis, Mycobacterium smegmatis, Mycobacterium bovis BCG, Escherichia coli, Bacteroides* (including *Porphyromonas*), *Pseudomonas aeruginosa, Helicobacter pylori, Brucella melitensis Campylobacter jejuni, Actinobacillus actinomycetemcomitans, Xenorhabdus nematophilus, Moraxella catarrhalis,* or *Borrelia burgdorferi.*

Particularly preferred bacteria are selected from at least one of: *S. sonnei, S. flexneri, Salmonella* bacterium, and *meningococcus.*

Virulent *Shigella* strains possess a 220kb plasmid that mediates virulence properties. This "virulence plasmid" has been shown to encode the genes for several aspects of *Shigella* virulence, including adhesins for target epithelial cells, the invasion plasmid antigens, virF, virG, and the like. A *Shigella* used with the invention may or may not possess a virulence plasmid. Absence of the plasmid can stabilise the strain during industrial culture, attenuate the strain by removing virulence factors (thereby increasing safety of manufacture), avoid the presence of the ShET-2 enterotoxin (encoded by the ospD3 or sen gene on the plasmid), and avoid the presence of msbB2 which is a second copy of the msbB gene responsible for acylation of lipid A.

As far as *Salmonella* bacterium is concerned, a particularly preferred strain is selected from: *Salmonella* Typhimurium, *Salmonella* Enteritidis and *Salmonella* Paratyphi A.

*Meningococcus* bacteria nOMVs are also preferred. Such vesicles can be prepared from any *meningococcal* strain. The vesicles are preferably prepared from a serogroup B strain, but it is also preferred to prepare them from serogroups other than B, such as one of: A, C, W135, Y or X. The strain may be of any serotype (*e.g.* 1, 2a, 2b, 4, 14, 15, 16, etc.), any serosubtype (*e.g.* P1.4), and any immunotype (*e.g.* L1; L2; L3; L3,7; L3,7,9; L10; etc.). The *meningococci* may be from any suitable lineage, including hyperinvasive and hypervirulent lineages, preferably any of the following seven hypervirulent lineages: subgroup I; subgroup III; subgroup IV-1; ET-5 complex; ET-37 complex; A4 cluster; lineage 3. Most preferably, nOMVs are prepared from the strain NZ98/254, or another strain with the P1.4 PorA serosubtype.

In another embodiment, bacteria for preparing nOMVs useful for the invention may be mutant strains which have been manipulated *e.g.* to enhance vesicles production, to express one or more desired antigen(s), and/or to knockout or modify an undesired gene (*e.g.* one which encodes a toxin or which encodes an enzyme involved in generating a toxic product, such as endotoxin).

In this direction, other preferred nOMVs for the invention are produced by a *Salmonella* bacterium, particularly a *S.* Typhimurium (also known as *Salmonella enterica serovar* Typhimurium) which does not express a functional ToIR protein.

Where the vesicles are prepared from *E.coli, Shigella* or *Salmonella* the bacterium may express no more than 4 of ToIA, ToIB, ToIQ, ToIR and Pal proteins. Thus at least one protein from the natural five-protein Tol-Pal system may be absent, resulting in a bacterium which, during growth in culture medium, releases greater quantities of outer membrane vesicles into the medium than the same bacterium expressing all 5 Tol-Pal proteins. Preferably ToIR is not expressed, but the other four proteins may be expressed (*i.e.* a ΔToIR strain).

In preferred embodiments, at least one of the five Tol-Pal proteins in *E.coli, Shigella* or *Salmonella* is removed *e.g.* by deletion or inactivation of the gene encoding the protein. Thus the bacterium may express 0, 1, 2, 3 or 4 of TolA, ToIB, ToIQ, ToIR and Pal proteins. Removal of one of the five proteins can suffice, in which case the bacterium expresses only 4 of these proteins. Preferably the ToIR protein is removed *e.g.* by inactivation of a starting strain's toIR gene. Thus a preferred bacterium may be tolA+ tolB+ tolQ+ ToIR- Pal+.

In some embodiments, the bacterium expresses all five Tol-Pal proteins, but at least one is mutated to cause hyperblebbing. For instance, the TolA, ToIQ, ToIR and/or Pal protein may be mutated such that the protein retains its membrane localisation but its interactions with other members of the Tol-Pal system are disrupted. The bacterium will thus retain TolA, ToIQ and ToIR as transmembrane proteins in the inner membrane, and Pal protein as a periplasm-facing lipoprotein in the outer membrane, but at least one of the TolA, ToIQ, ToIR and/or Pal proteins is mutated and not fully functional.

In addition, other mutations may also be present *e.g.* to give OAg-deficient strains, such as ΔgaIU, ΔgaIE or ΔwbaP in *E.coli, Shigella* or *Salmonella* strains.

In one further preferred embodiment, a *meningococcus* does not express a functional MItA protein. Knockout of MItA (the membrane-bound lytic transglycosylase, also known as GNA33) in *meningococcus* provides bacteria which spontaneously release large amounts of nOMVs into culture medium, from which they can be readily purified. For instance, the vesicles can be purified using the two stage size filtration process, comprising: (i) a first filtration step in which vesicles are separated from the bacteria based on their different sizes, with the vesicles passing into the filtrate; and (ii) a second filtration step in which the vesicles are retained in the retentate.

In the present invention, it is preferred that -OAg is present on the nOMVs because it has been observed (*e.g.* nOMVs from *Salmonella* and *Shigella*) that, the presence of the -OAg on the surface of said nOMVs is advantageous in providing a bivalent vaccine, as the -OAg can act as a protective antigen. Some preferred strains have penta- or tetra-acylated less toxic LPS, which includes attached -OAg, after the mutation of msbB, htrB, ddg and/or PagP (see *e.g.* Rossi O et al, Clin Vaccine Immunol. 2016 Apr 4;23(4):304-14 and Rossi O. et al, J. Biol. Chem. 2014 Sep 5;289(36):24922-35).

In *Neisseria,* the strain has preferably a modified fur gene. According to this embodiment, mutant *Neisseria* are engineered to reduce or switch off expression of at least one gene involved in rendering toxic the lipid A portion of LPS, in particular of Ipxl1 gene. In this way, the resulting nOMVs present a reduced toxicity respect to the wild type strain, since the conversion of acylated lipid A in a less acylated form.

Similarly, preferred mutant *Neisseria* for the invention are engineered to reduce or switch off expression of at least one gene involved in the capsular saccharide synthesis or export, in particular of synX and/or ctrA genes. In this way, the resulting nOMVs may present a cross protection versus different serotypes, particularly appreciated by the skilled in the art.

In preferred embodiments a strain may include one or more of the knockout and/or hyper-expression mutations disclosed *e.g.* in Fukusawa et al. (1999), Vaccine 17:2951-2958. For instance, following the therein guidance and nomenclature, useful genes for down-regulation and/or knockout include: (a) Cps, CtrA, CtrB, CtrC, CtrD, FrpB, GalE, HtrB/MsbB, LbpA, LbpB, LpxK, Opa, Opc, PilC, PorB, SiaA, SiaB, SiaC, SiaD, TbpA, and/or TbpB; (b) CtrA, CtrB, CtrC, CtrD, FrpB, GalE, HtrB/MsbB, LbpA, LbpB, LpxK, Opa, Opc, PhoP, PilC, PmrE, PmrF, SiaA, SiaB, SiaC, SiaD, TbpA, and/or TbpB; (c) ExbB, ExbD, rmpM, CtrA, CtrB, CtrD, GalE, LbpA, LpbB, Opa, Opc, PilC, PorB, SiaA, SiaB, SiaC, SiaD, TbpA, and/or TbpB; or (d) CtrA, CtrB, CtrD, FrpB, OpA, OpC, PilC, PorB, SiaD, SynA, SynB, SynX and/or SynC.

As above mentioned, the nOMVs are covalently linked to the bifunctional linker by way of at least one protein residue, generally located on the surface of the vesicle. In this direction, the proteins will preferably react with a terminal end of the linker by means of one or more amino, thiol or hydroxyl amino acid functionalities, being this latter an alpha hydroxyl group or part of the carboxy aminoacid functionality. Preferably, the protein functional group is an amino group, more preferably a primary amine (-NH₂). These functional groups may naturally be present in the amino acid portions of interest, or even introduced artificially for the purposes of conjugation.

The selected linker is a homobifunctional bivalent linker: therefore, the protein functional group and the antigen functional group that will respectively react with the terminal portions of the linker will be preferably the same. By way of example, a lysine aminoacid residue of one or more nOMV proteins will react with the linker (*e.g.* BS3) via the corresponding -NH₂ functional group. In the same way, also a selected antigen (*e.g.* Pfs25) will react with the remaining free terminal portion of the linker via the relevant amino (-NH₂) groups. Of note, and as well explained in the present description, the reaction occurs without substantial cross reaction or aggregation formation, thus leading to the final product, useful *e.g.* for the preparation of multivalent vaccines, in a very reliable and versatile way, and differently from using dOMVs, as indicated in the comparative Example 8 and 8a.

Preferred amino acid residues include, arginine, lysine, asparagine, glutamine, aspartic or glutamic acid, cysteine and histidine. Preferably, the nOMV proteins are those having one or more aminoacid moiety showing free amino groups, preferably-NH₂ groups. Even more preferably said aminoacid moiety is the arginine and/or lysine, whereby different -NH₂ groups form different arginine and/or lysine proteins are able to selectively react with the linker according to the present invention.

As far as the divalent homobifunctional linker is concerned, this is typically a molecule of a certain length, with a suitable water solubility and polarity, able to covalently bind the nOMV proteins and the antigen by its terminal ends respectively. In order to optimize the solubility of the chosen linker, it may be expedient to introduce one or more polar group such as sulfate, sulfite, phosphate and the like, or even use the corresponding salt thereof, *e.g.* as alkaline or alkaline earth metal salt, where possible. Due to the versatility of the present invention, it is possible to use different linkers, in terms *e.g.* of lengths, polarity, and steric hindrance, thus providing a covalent bond with both the nOMV protein residues and the selected antigen. By that, the invention allows the preparation of a series of final nOMV-linker-antigen conjugates endowed with remarkable and specific behavior, with particular regard to their immunogenicity and activity.

The linker is homobifunctional (*i.e.* having equal terminal functionalities). Even more preferably, the liker is symmetric with respect to a hypothetical vertical axis.

Thus the divalent linker according to the present invention has a general formula **(I):**

X-L-X' **(I)**

wherein:
- X and X': are the same, and are a functional group able to selectively react with nOMV proteins on one hand and with the selected antigen on the other hand, preferably by forming ester, amido or thioester moieties;
- -L-: is a divalent linear or branched C₁-C₁₅ alkyl or alkenyl group optionally substituted, and optionally interrupted by one or more heteroatom selected from: oxygen (-O-), sulfur (-S-), nitrogen (-NH- or optionally substituted -N- group)

In one embodiment, -L- is preferably a divalent linear C₃-C₁₂ alkyl group, optionally substituted or interrupted by one or more oxygen (-O-) heteroatom. In a still more preferred embodiment, - L- is a divalent linear C₃-C₆ alkyl group.

According to formula (I), the linker is further characterized by having both terminal portions bearing two functionalities X and X' which are the same thus providing a divalent homo-functional linker. In one embodiment, the X and/or X' groups can be any which form esters, thioester or amide when combined with a hydroxyl, thiol or amino functionality respectively.

Preferably, X and/or X' are N-hydroxysuccinimide ester conjugates, more preferably selected from at least one of: 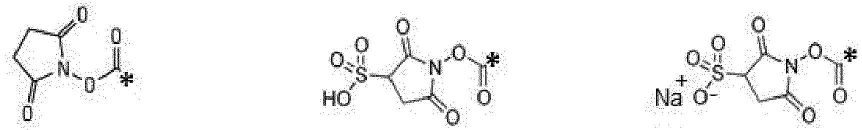 wherein the * represents the point of contact with the -L- spacer in formula (I), as above defined. Thus, in a still preferred embodiment, the linker is selected from at least one of: disuccinimidyl glutarate (DSG), disuccinimidyl suberate (DSS), hydroxysuccinimide, N oxysuccinimide and adipic acid N-hydroxysuccinimide diester (SIDEA) and Bis(sulfosuccinimidyl) suberate (BS3, CAS No. 82436-77-9).

In one embodiment, additional preferred bifunctional linkers reactive with amines for use with the invention are selected from at least one of: ethylene glycol bis[succinimidylsuccinate], bis(sulfosuccinimidyl)tri(ethylene glycol) (BS(PEG)3), bis(sulfosuccinimidyl)tetra(ethylene glycol) (BS(PEG)4), bis(sulfosuccinimidyl)penta(ethylene glycol) (BS(PEG)5) and bis(sulfosuccinimidyl)exa(ethylene glycol) (BS(PEG)6), where bis(sulfosuccinimidyl)penta(ethylene glycol) (BS(PEG)5, CAS No 756526-03-1) is particularly preferred.

Preferred homobifunctional linkers able to react with thiol functional groups on nOMV protein and antigen according to the invention, are those having X and X' selected from at least one of: 2-pyridyldithio, maleimide or iodoacetyl residue.

Another linker suitable for the reaction with the nOMV protein hydroxyl group as above defined, is Adipic acid dihydrazide (ADH).

Preferred linkers are selected from at least one of: (BS(PEG)5), Disuccinimidyl glutarate (DSG) or a salt thereof, SIDEA and BS3, where BS3 is even more preferred (for a general reference on BS3 see *e.g.* US patent 4,965,338). According to a still preferred embodiment, DSG is particularly useful when operating at pH of about 9. Surprisingly, the efficacy of the conjugation reaction can be conveniently increased when (BS(PEG)5) or BS3 are used as divalent linker, substantially in the absence of vesicle aggregates formation. In this respect, it has to be highlighted that the use of BS3 according to the present invention does not provide substantial crosslinking of nOMV surface proteins to form high-molecular-mass aggregates, but rather, selective reaction with nOMV on one terminal end, and with the selected antigen on the other end. This behavior is further supported by the herein enclosed experimental part, where example 8 and example 8a (comparative, using dOMV) are described.

Advantageously, any proper antigen may be conjugated to the nOMV to obtain the nOMV-linker-antigen conjugates of the invention. In any case, the connection of one or more selected antigen produces an immunogenic conjugate which can raise an immune response which recognises said antigen, and which also recognises one or more components in the nOMV, thereby conveniently providing a multivalent vaccine. Antigens will be included in the present conjugates at a concentration which is high enough to elicit, when administered to a host, an immune response which recognises that antigen. Moreover, the immune response is preferably protective against the pathogen from which the antigen was derived, even more preferably against one of the pathogens listed below.

In one embodiment of the invention, the nOMV is conjugated to at least one homologous antigen, *i.e.* derived from the same organism from which the nOMV is derived. In a still preferred embodiment, the selected antigen is a heterologous antigen *i.e.* derived from a different organism from the organism from which the nOMV is derived.

In any case, the antigens may generally be selected from any immunogenic polypeptides, *i.e.* polypeptides able to elicit an immune response when administered to a subject. Polypeptides used with the invention will include an amino acid having a residue, or a side chain, with a functional group suitable for conjugation, preferably an amino or a thiol group, even more preferably of general formula: -NH₂ or -SH. These residues may naturally be present in an antigen, or they may be introduced artificially for the purposes of conjugation. Preferred amino acid residues include, but are not limited to: arginine, lysine, asparagine, glutamine, cysteine and histidine. The most preferred amino acid residue for conjugation is lysine.

Polypeptide antigens conjugated to nOMVs are preferably prepared in substantially pure or substantially isolated form (*i.e.* substantially free from other polypeptides). They can be prepared by various means *e.g.* by chemical synthesis (at least in part), by digesting longer polypeptides using proteases, by translation from RNA, by purification from cell culture (*e.g.* from recombinant expression or from native culture), and the like. Recombinant expression in an *E.coli* host is a useful expression route. Polypeptide antigens can take various forms (*e.g.* native, fusions, glycosylated, non-glycosylated, lipidated, disulfide bridges and the like). Polypeptide antigens used with the invention have a preferred average molecular weight of at least 1 kDa, more preferably of at least 3.5 kDa, even more preferably from 10 to 80 kDa. Still more preferably, the average molecular weight is comprised from 15 to 75 kDa.

Further preferred polypeptide antigens for conjugating to nOMVs according to the present invention comprise an epitope from a fungal, bacterial, protozoan or viral polypeptide. Preferred protozoan polypeptides are from a *Plasmodium* (such as *P. falciparum, P. vivax, P. ovale*). Particularly preferred bacterial polypeptides are selected from: *E*. *coli, N. meningitidis,* and *Streptococci* (such as *S.agalactiae, S.pneumoniae, S.pyogenes*).

Preferred *E.coli* polypeptide antigens include CTF1232 (SEQ ID NO: 14), 3526 (SsIE, SEQ ID NO: 15) and 405 (FdeC, SEQ ID NO: 16). As a preferred example, nOMV from *Shigella* or *Salmonella* can be conjugated to CTF1232, 3526 or 405, according to the present invention, to generate a bivalent vaccine covering both enterotoxigenic *E. coli* (ETEC) and *Shigella*/*Salmonella.*

In one embodiment, the considered *N.meningitidis* polypeptides are able, when administered to a mammal, to elicit an antibody response that is bactericidal against *meningococcus.* Preferred *N.meningitidis* polypeptides for use with the invention are selected from at least one of: NHBA, NadA, NsPA, NhhA, App and fHbp, as herein below detailed.

### NHBA antigen.

The NHBA antigen was included in the published genome sequence for meningococcal serogroup B strain MC58 as gene NMB2132 (GenBank accession number GI:7227388; SEQ ID NO: 2 herein). The sequences of NHBA antigen from many strains have been published since then. Various immunogenic fragments of the NHBA antigen have also been reported. Preferred NHBA antigens for use with the invention comprise an amino acid sequence: (a) having 50% or more identity (*e.g.* 60%, 65%, 70%, 75%, 80%, 85%, 90%, 91%, 92%, 93%, 94%, 95%, 96%, 97%, 98%, 99%, 99.5% or more, *e.g.* 100%) to SEQ ID NO: 2; and/or (b) comprising a fragment of at least 'n' consecutive amino acids of SEQ ID NO: 2, wherein 'n' is 7 or more (*e.g.* 8, 10, 12, 14, 16, 18, 20, 25, 30, 35, 40, 50, 60, 70, 80, 90, 100, 150, 200, 250 or more). Preferred fragments of (b) comprise an epitope from SEQ ID NO: 2. The most useful NHBA antigens of the invention can elicit antibodies which, after administration to a subject, can bind to a meningococcal polypeptide consisting of amino acid sequence SEQ ID NO: 2. Advantageous NHBA antigens for use with the invention can elicit bactericidal *anti-meningococcal* antibodies after administration to a subject.

### NadA antigen.

The NadA antigen was included in the published genome sequence for *meningococcal* serogroup B strain MC58 (see *e.g.* Tettelin et al. (2000) Science 287:1809-1815) as gene NMB1994 (GenBank accession number GI:7227256; SEQ ID NO: 3 herein). The sequences of NadA antigen from many strains have been published since then, and the protein's activity as a *Neisseria*l adhesin has been well documented. Various immunogenic fragments of NadA have also been reported. Preferred NadA antigens for use with the invention comprise an amino acid sequence: (a) having 50% or more identity (*e.g.* 60%, 65%, 70%, 75%, 80%, 85%, 90%, 91%, 92%, 93%, 94%, 95%, 96%, 97%, 98%, 99%, 99.5% or more, e.g. 100%) to SEQ ID NO: 3; and/or (b) comprising a fragment of at least 'n' consecutive amino acids of SEQ ID NO: 3, wherein 'n' is 7 or more (e.g. 8, 10, 12, 14, 16, 18, 20, 25, 30, 35, 40, 50, 60, 70, 80, 90, 100, 150, 200, 250 or more). Preferred fragments of (b) comprise an epitope from SEQ ID NO: 3. The most preferred NadA antigens of the invention can elicit antibodies which, after administration to a subject, can bind to a *meningococcal* polypeptide consisting of amino acid sequence SEQ ID NO: 3. Advantageous NadA antigens for use with the invention can elicit bactericidal *anti-meningococcal* antibodies after administration to a subject. SEQ ID NO: 7 is one such fragment.

### NspA antigen.

The NspA antigen was included in the published genome sequence for *meningococcal* serogroup B strain MC58 (see *e.g.* Tettelin et al. (2000) Science 287:1809-1815) as gene NMB0663 (GenBank accession number GI:7225888; SEQ ID NO: 4 herein). The sequences of NspA antigen from many strains have been published since then. Various immunogenic fragments of NspA have also been reported. Preferred NspA antigens for use with the invention comprise an amino acid sequence: (a) having 50% or more identity (*e.g.* 60%, 65%, 70%, 75%, 80%, 85%, 90%, 91%, 92%, 93%, 94%, 95%, 96%, 97%, 98%, 99%, 99.5% or more, e.g. 100%) to SEQ ID NO: 4; and/or (b) comprising a fragment of at least 'n' consecutive amino acids of SEQ ID NO: 4, wherein 'n' is 7 or more (*e.g.* 8, 10, 12, 14, 16, 18, 20, 25, 30, 35, 40, 50, 60, 70, 80, 90, 100, 150, 200, 250 or more). Preferred fragments of (b) comprise an epitope from SEQ ID NO: 4. The most preferred NspA antigens of the invention can elicit antibodies which, after administration to a subject, can bind to a meningococcal polypeptide consisting of amino acid sequence SEQ ID NO: 4. Advantageous NspA antigens for use with the invention can elicit bactericidal *anti-meningococcal* antibodies after administration to a subject.

### NhhA antigen.

The NhhA antigen was included in the published genome sequence for *meningococcal* serogroup B strain MC58 (see *e.g.* Tettelin et al. (2000) Science 287:1809-1815) as gene NMB0992 (GenBank accession number GI:7226232; SEQ ID NO: 5 herein). The sequences of NhhA antigen from many strains have been published since *e.g.* WO00/66741 and WO01/55182, and various immunogenic fragments of NhhA have been reported. It is also known as Hsf. Preferred NhhA antigens for use with the invention comprise an amino acid sequence: (a) having 50% or more identity (*e.g.* 60%, 65%, 70%, 75%, 80%, 85%, 90%, 91%, 92%, 93%, 94%, 95%, 96%, 97%, 98%, 99%, 99.5% or more, e.g. 100%) to SEQ ID NO: 5; and/or (b) comprising a fragment of at least 'n' consecutive amino acids of SEQ ID NO: 5, wherein 'n' is 7 or more (*e.g.* 8, 10, 12, 14, 16, 18, 20, 25, 30, 35, 40, 50, 60, 70, 80, 90, 100, 150, 200, 250 or more). Preferred fragments of (b) comprise an epitope from SEQ ID NO: 5. The most preferred NhhA antigens of the invention can elicit antibodies which, after administration to a subject, can bind to a *meningococcal* polypeptide consisting of amino acid sequence SEQ ID NO: 5. Advantageous NhhA antigens for use with the invention can elicit bactericidal *anti-meningococcal* antibodies after administration to a subject.

### App antigen.

The App antigen was included in the published genome sequence for *meningococcal* serogroup B strain MC58 (see *e.g.* Tettelin et al. (2000) Science 287:1809-1815) as gene NMB1985 (GenBank accession number GI:7227246; SEQ ID NO: 6 herein). The sequences of App antigen from many strains have been published since then. Various immunogenic fragments of App have also been reported. Preferred App antigens for use with the invention comprise an amino acid sequence: (a) having 50% or more identity (*e.g.* 60%, 65%, 70%, 75%, 80%, 85%, 90%, 91%, 92%, 93%, 94%, 95%, 96%, 97%, 98%, 99%, 99.5% or more, e.g. 100%) to SEQ ID NO: 6; and/or (b) comprising a fragment of at least 'n' consecutive amino acids of SEQ ID NO: 6, wherein 'n' is 7 or more (*e.g.* 8, 10, 12, 14, 16, 18, 20, 25, 30, 35, 40, 50, 60, 70, 80, 90, 100, 150, 200, 250 or more). Preferred fragments of (b) comprise an epitope from SEQ ID NO: 6. The most preferred App antigens of the invention can elicit antibodies which, after administration to a subject, can bind to a *meningococcal* polypeptide consisting of amino acid sequence SEQ ID NO: 6. Advantageous App antigens for use with the invention can elicit bactericidal *anti-meningococcal* antibodies after administration to a subject.

### fHbp antigen.

The factor H binding protein exists as three variants (v1, v2 and v3), and the invention can use any of these as preferred embodiment.

A v1 fHbp preferably comprises (a) an amino acid sequence which has at least k'% identity to SEQ ID NO: 8, and/or (b) a fragment of SEQ ID NO: 8. k' refers to percentage identity and could be defined as any number from 1 to 100. With reference to amino acid or nucleic acid sequences, generally the identity used in the application starts from as low as 40% with specific references to higher percentages, e.g. 60%, 65%, 70%, 75%, 80%, 85%, 90%, 91%, 92%, 93%, 94%, 95%, 96%, 97%, 98%, 99%, 99.5% or more, e.g. 100%.

The fragment will preferably include at least one epitope from SEQ ID NO: 8. Preferably, the v1 fHbp can elicit antibodies which are bactericidal against v1 strains *e.g.* against strain MC58 (available from the ATCC as 'BAA-335').

A v2 fHbp preferably comprises (a) an amino acid sequence which has at least k'% identity to SEQ ID NO: 1, and/or (b) a fragment of SEQ ID NO: 1. Information about 'k' and fragments are given above. The fragment will preferably include at least one epitope from SEQ ID NO: 1. Preferably, the v2 fHbp can elicit antibodies which are bactericidal against v2 strains *e.g.* against strain M2091 (ATCC 13091).

A v3 fHbp preferably comprise (a) an amino acid sequence which has at least k'% identity to SEQ ID NO: 9, and/or (b) a fragment of SEQ ID NO: 9. Information about 'k' and fragments are given above. The fragment will preferably include at least one epitope from SEQ ID NO: 9. Preferably, the v3 fHbp can elicit antibodies which are bactericidal against v3 strains *e.g.* against strain M01-240355.

Antigens from Group A *Streptococcus* (GAS), Group B *Streptococcus* (GBS) and *Pneumococcus* are also equally preferred. As non-limiting examples, GAS25 (Slo), GAS40 (SpyAD) and GAS57 (SpyCEP) antigens can be incorporated into conjugates in accordance with some embodiments of the invention.

Plasmodium antigens are further preferred. These can be from any suitable species, where preferred species are selected from: *P.falciparum, P.vivax* and *P.ovale.*

Still another preferred antigen is Pfs25 (SEQ ID NO: 10), which is a sexual stage antigen of *P.falciparum* expressed on the surface of zygote and ookinete forms of the parasite. Another preferred antigen is Pfs48/45, which is a transmission-blocking vaccine candidate. Recently the C-terminal 10 cysteine fragment (10C) of Pfs48/45, containing three known epitopes for transmission blocking antibodies, has been produced as a chimera with the N-terminal portion of GLURP (RO), the asexual blood-stage antigen glutamate-rich protein. The resulting fusion protein (RO10C) elicited high levels of transmission-blocking antibodies in rodents (see Theisen et al. (2014) Vaccine 32:2623-2630). Shing et al. (2015) Vaccine 33:1981-1986 describes a chimera containing truncated 6C-fragments, which increases the yield of correctly-folded conformer. The RO6C construct was able to elicit high titer transmission blocking antibodies in rats. RO6C (SEQ ID NO: 11) is a preferred antigen that can be conjugated according to the present invention.

Another preferred antigen is the *circumsporozoite* protein (CSP; SEQ ID NO: 12).

Shorter peptides from CSP may also be conjugated according to the present invention. For example, the 12 amino acid (NANP)₃ peptide (SEQ ID NO: 13) derived from CSP can be used according to preferred embodiments.

In another still preferred embodiment the antigens are a saccharide species. The invention is in fact also suitable for conjugating one or more selected saccharide antigens to nOMVs, whereby saccharides may be used in their full-length natural form. As an alternative, a particular size fraction can also advantageously be selected. Thus, saccharides may be fragmented from their natural length, and optionally a size fraction of these fragments can be used. Even further, the saccharides are not limited to saccharides purified from natural sources and synthetic or semi-synthetic saccharides can be used instead. The polysaccharide antigen to be used according to the invention is generally functionalized in order to allow the reaction with the divalent linker. Thus, in one embodiment, when the antigen is a polysaccharide, the invention comprises the oxidation of the anomeric hydroxyl group of said antigen to aldehyde, followed by reductive amination conversion to amino group, e.g. using NalO₄ and NaBH₄, according to procedures known in the art. In this way, the polysaccharide antigen will present a suitable - NH₂ group for the reaction with the terminal portion of the linker, already connected to the nOMV to give nOMV-Linker-antigen conjugate.

Preferred saccharide antigens are bacterial capsular saccharides (CPSs). These include, but are not limited to, the capsular saccharides selected from at least one of: *Haemophilus influenzae* type B; *Neisseria meningitidis* serogroups A, C, W135, X and Y, where serogroups A and C are particularly preferred; *Streptococcus pneumoniae* serotypes 1, 2, 3, 4, 5, 6A, 6B, 7F, 8, 9N, 9V, 10A, 11A, 12F, 14, 15B, 17F, 18C, 19A, 19F, 20, 22F, 23F, and 33F; *Salmonella* including *Salmonella enterica serovar* Typhi Vi, either full length or fragmented (indicated as fVi); *Streptococcus agalactiae* serotypes Ia, Ib, and III; Streptococcus pyogenes, *Shigella* sp.

In a preferred embodiment, the nOMV is a GMMA from *Meningococcus* bacteria, preferably prepared from a serogroup B strain, and the selected antigen is a capsular saccharide from *Neisseria meningitidis* serogroups A or C, even more preferably conjugated to the GMMA via a SIDEA linker.

In any case, and as above mentioned, the selected antigens could be conjugated to nOMVs derived from the same or even from a different bacterial strain, thus providing a multivalent vaccine. In this respect, in a more preferred embodiment of the invention, the nOMV and the saccharide antigen are derived from different bacterial strains.

Other preferred saccharide antigens are β-glucans, particularly useful for protecting against *C.albicans* (for a general reference see Sandlin et al. (1995) Infect. Immun., 63:229-37).

Other preferred saccharide antigens are poly-rhamnose oligosaccharides for protecting against Group A *Streptococcus* (GAS). Native GAS saccharide has a poly-rhamnose backbone substituted with NAcGlcN. Synthetic oligosaccharides of poly-rhamnose, or oligomers with the structure of native GAS saccharide, can be conjugated to nOMVs according to the invention.

The conjugates of the invention are immunogenic, as demonstrated by the studies in mice and supported by the herein included experimental part.

As above set forth, in a further aspect, the invention refers to a process for preparing nOMV-linker-antigen conjugates, comprising the reaction of at least a nOMV protein residue with a first terminal portion of a divalent linker, followed by reaction of the second terminal portion of such linker with a selected foreign antigen, as herein below described in more details.

Thus, according to an embodiment, the invention refers to a process for the preparation of nOMV-linker-antigen conjugates, comprising the step of:
(i) reacting at least one protein on the nOMV surface with a divalent linker to form a nOMV-linker intermediate; and
(ii) reacting the thus obtained nOMV-linker intermediate with at least a foreign antigen to form the nOMV-linker-antigen conjugates of the invention.

According to an embodiment, the nOMVs are initially suspended in a proper buffer, such as MES (2-(N-morpholino)ethanesulfonic acid) buffer, borate buffer or PBS (phosphate buffer saline), in order to select a pH comprised from 5 to 9, preferably from 5 to 7 or from 7 to 9, depending for instance on the selected linker or conjugation conditions. Same pH ranges are also used for the step ii) for the reaction between the free terminal end of the linker and the selected antigen as herein below described in details.

After step i) the percentage of nOMV functionalization is comprised from 15% to 60%, mainly depending on the kind of divalent linker used. To his regards, the % of reactive functional groups at the free terminal end of the linker is from 15% to 40%, preferably from 30% to 35%, depending on the kind and stability of said reactive functional groups. It is in fact noticed that such ranges allow for an implemented efficacy of the process, thus resulting in higher amount of final antigen conjugate of the invention.

The thus obtained buffered suspension has a nOMV concentration comprised from 2 and 10 mg/mL, preferably from 3 to 6 mg/mL. The chosen linker, is generally added in amounts depending *e.g.* on the -NH₂ groups on the nOMV, preferably in excess, even more preferably comprised from 10 to 20 equivalents per mole of -NH₂.

Depending on the linker, it could be convenient to preventively solve it in a polar dry solvent, such as DMSO or the like, in order to facilitate the handling and the efficacy, thus obtaining improved results in terms of overall yield and reproducibility.

The mixture is then incubated at room temperature (*e.g.* from about 15 to 40° C) for a period of time generally from 30 minutes to 4 hours. Subsequently, the thus obtained nOMV-linker intermediate is purified, *e.g.* by ultracentrifugation, and then reacted with the selected antigen according to step ii). The antigen is generally solved in a proper buffer solution, such as phosphate buffered saline. The antigen is preferably added in an amount ranging from 2:1 to 1:2 w/w ratio over the nOMV-linker intermediate, or more preferably in a 1:1 ratio. The reaction can be monitored *e.g.* by HPLC/SEC and the final product formation can be confirmed by SDS page/western blot analysis.

As an alternative embodiment, the conjugation reaction comprises the steps of: (i) reacting the antigen with a linker to form an antigen-linker intermediate; and (ii) reacting at least one protein on the nOMV with the thus formed antigen-linker intermediate to form a nOMV-linker-antigen conjugate.

As another alternative, the conjugation reaction comprises the steps of: (i) reacting the antigen with a first linker to form an antigen-linker intermediate; (ii) reacting at least one protein on the nOMV with a second linker to form a nOMV-linker intermediate; and (iii) reacting the antigen-linker with the nOMV-linker to form a nOMV-linker-antigen conjugate.

The skilled person will understand that when a homobifunctional linker is used, the protein and the antigen functional groups involved in the reaction will be the same chemical entity, as above explained in details.

Thus, according to one preferred embodiment, the process of the present invention comprises the steps of:
(i) reaction of a -NH₂ group of at least a nOMV protein with a divalent homobifunctional linker to form a nOMV-linker intermediate; and
(ii) reaction of said intermediate with a -NH₂ group on a selected foreign antigen to form nOMV-linker-antigen conjugate of the invention.

This type of conjugation reaction is illustrated in Scheme 2 below, using, by way of example, a GMMA as native vesicle and BS3 as the linker.

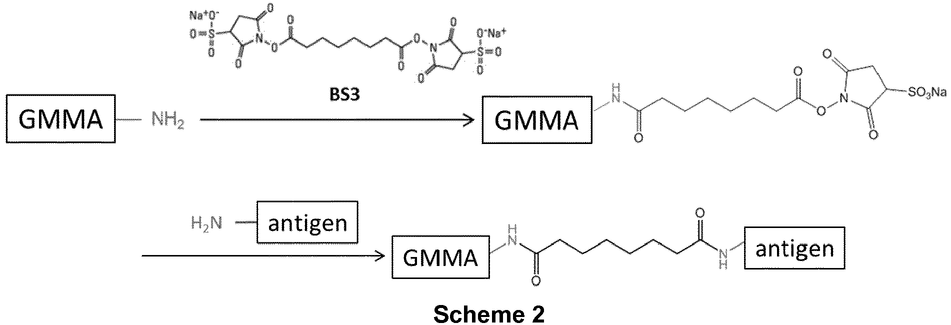

Where the linker reactions with the protein on the surface of the nOMV and the antigen involve different functional groups (such as an amine on the protein on the vesicle and a thiol on the antigen, or vice versa) it is preferred to use a heterobifunctional linker of the above general formula (I) X-L-X', where X and X' are different to each other, as above defined, and L is a moiety as above defined. The X group can react with one functional group, *e.g.* an amine on the nOMV protein; whereas X' group can react with a different functional group, *e.g.* a thiol on the selected antigen. Preferably, the X group is N-hydroxysuccinimide or N-oxysuccinimide, whereas the X' group is selected from at least one of: 2-pyridyldithio, maleimide or iodoacetyl group.

An alternative conjugation process of the invention includes reacting the antigen with a first linker and a protein on the vesicle with a second linker, then reacting the first and second linker together to form the conjugate.

By way of example, either the antigen or the protein on the nOMV may be reacted with a linker terminating in a maleimide group, for instance by reacting a primary amine or either the antigen or the protein on the nOMV with succinimidyl-4-(N-maleimidomethyl)cyclohexane-1-carboxylate (SMCC) or N-(γ-maleimidobutyryloxy)succinimide ester (GMBS). A thiol on either the antigen or the protein on the nOMV may then be reacted with the maleimide. The thiol may be native to the protein on the nOMV or antigen or the thiol may be the result of reacting the protein on the nOMV or antigen with a separate linker. This type of conjugation reaction is exemplified in Scheme 3 below, using, by way of example, GMMA and fHbp as the antigen:

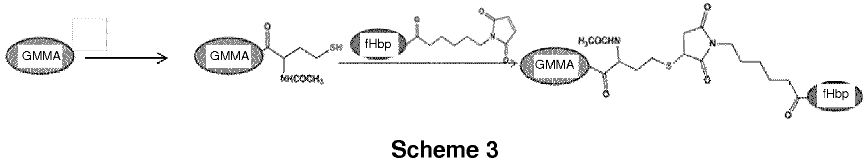

Advantageously, due to its versatility, the invention can be used for the preparation of a variety of conjugates, particularly appreciated by the skilled person when faced with the problem to find convenient and reliable methodologies for obtaining immunogenic conjugates.

As a further alternative, a protein on the vesicle may be linked to the antigen by (i) modifying the vesicle protein to include an alkyne; (ii) modifying the antigen to include an azide, then (iii) reacting the alkyne and azide, known as "click chemistry", as exemplified in Example 6. Alternatively, the antigen may be modified to include an alkyne and the vesicle may be modified to include an azide. This type of conjugation reaction is illustrated in Scheme 4 below, using, by way of example, GMMA as the vesicle and fHbp as the antigen and using copper free click chemistry. Only homobifunctional linkers according to the claims are part of the invention.

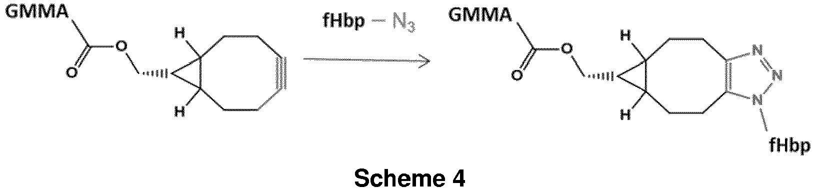

The present invention is also useful for the preparation of variously functionalized nOMVs, allowing multivalent presentation of different antigens on the surface of the selected vesicle.

Thus, according to a preferred embodiment, the invention refers to an immunogenic conjugate comprising a nOMV as above set forth, having at least a protein moiety connected to a first antigen via a divalent linker, wherein said first antigen is connected to a second different antigen. In this direction, the two selected antigens (herein indicated as Ag1 and Ag2) may be antigen. In this direction, the two selected antigens (herein indicated as Ag1 and Ag2) may be coupled together to give an Antigen 1-Antigen2 derivative (Ag1-Ag2), which can be subsequently connected to the selected nOMV surface protein moiety by a suitable divalent linker as above described, to give a conjugate indicated by the general formula (I):

nOMV-linker-Ag1-Ag2 **(I)**

Alternatively, the nOMV protein residue can first be connected to the selected Ag1 via a linker as above described, and subsequently a second Ag2 is further connected to said Ag1 residue, to give the conjugates of the above general formula (I).

In any case, preferred nOMV are the GMMA vesicles. The Ag1 and Ag2 in the formula (I) can be selected among the preferred antigens as above described, either protein or polysaccharide moieties. Preferably, the antigens used for the multi functionalization as herein contemplated are both proteins or both polysaccharides.

According to a further embodiment, the present invention refers to an immunogenic conjugate comprising a nOMV, having at least a surface protein moiety connected to a first antigen (Ag1) by a divalent linker, and at least another surface protein moiety connected to a second different antigen (Ag2) by a divalent linker, to give a conjugate indicated by the general formula (II):

Ag1-linker-nOMV-linker-Ag2 **(II)**

As indicated in formula (II) Ag1 and Ag2 are each individually connected to the nOMV via a suitable linker. In this direction, the linker for the connection of the nOMV protein to the Ag1 or Ag2 can independently be the same or differ from each other.

The conjugates of formula (II) can advantageously provide selective multi functionalization of nOMV, preferably GMMA, by using a specific functionalization pattern. The Ag1 and Ag2 in the formula (II) can be selected among the preferred antigens as above described, either protein or polysaccharide moieties. Preferably, the antigens used for the multi functionalization as herein contemplated are both proteins or both polysaccharides.

Thus according to a more preferred embodiment, the invention refers to a conjugate of formula (II) wherein Ag1 comprises the capsular saccharide of *Nesseria mengitidis* serogroup A (MenA) and Ag2 comprises the capsular saccharide of *Nesseria mengitidis* serogroup C (MenC), even more preferably having the nOMV which is a GMMA vesicle, more preferably obtained from *Nesseria mengitidis* serogroup B, even more preferably expressing fHbp v2 and v3.

Even more preferably, the two linkers, each connecting Ag1 and Ag2, preferably MenA and MenC respectively, are the same, more preferably SIDEA. In this respect, the obtained multifunctionalized GMMA can be used as immunogenic agent against the Neisseria bacteria from MenB, MenA or MenC, as supported by the SBA data collected in the present experimental part.

In one embodiment, said conjugates are prepared by a process comprising adding a mixture of activated MenA and MenC polysaccharides to GMMA, according to the present procedure as above described. The MenA and MenC polysaccharides are preferably activated by reaction with SIDEA, thus providing suitable SIDEA-MenA and SIDEA-MenC derivatives, able to react with the -NH₂ groups of the nOMV protein through the terminal portion of the SIDEA moiety, thus leading to the conjugates of the above general formula (II).

Western blot and HPAEC-PAD analysis confirms the formation of the conjugates where the MenA and the MenC saccharides are randomly connected to the GMMA surface proteins via the linker, and no aggregation of nOMV is detected by dls.

The skilled person will recognize that in light of the versatility of the proposed technology, the present invention may be suitable used for the multi functionalization of nOMV, preferably GMMA, even with more than 2 different antigens. Beside the possibility of selecting different antigens as above indicated, the present invention also allows for the introduction of different amounts of different antigens, thus modulating the antigen/nOMV ratio according e.g. to the selected antigen or used nOMV.

Thus, according to additional embodiments, the invention refers to conjugates of general formula (II) wherein the Ag1 comprises Hib and Ag2 comprises Hia, where said Ag1 and Ag2 are each individually conjugated to a GMMA, preferably a pertussis GMMA, even more preferably by a BS3 linkers. In a still additional embodiments, the invention refers to conjugates of general formula (II) wherein the Ag1 comprises ETEC 405 (FdeC, Seq ID NO:16) and Ag2 comprises ETEC3526 (SsIE, Seq ID NO:15), where said Ag1 and Ag2 are each independently conjugated to a GMMA, preferably a Shigella Sonnei GMMA, even more preferably by a BS3 linker.

According to a further aspect, the invention refers to the conjugates of the invention for use as a medicament, particularly as immunogenic agent, even more preferably for one or more of the pathogens as herein indicated. In other words, the invention refers to the use of the present conjugates for the manufacture of an immunogenic composition.

According to a further aspect, the invention refers to an immunogenic composition, preferably a vaccine, comprising a conjugate of the invention and at least one additional pharmaceutically acceptable carrier, excipient or adjuvant. Generally, pharmaceutically acceptable carrier, excipient or adjuvant can be any substance that does not itself induce the production of antibodies harmful to the patient receiving the composition, and which can be administered. Pharmaceutically acceptable carriers and excipient are those used in the art, and can include liquids such as water, saline, glycerol and ethanol. Auxiliary substances, such as wetting or emulsifying agents, pH buffering substances, and the like, can also be present in such vehicles, according to the prior art.

The invention also provides a conjugate of the invention for use in a method for raising an immune response in a vertebrate, preferably a mammal, comprising administering a conjugate of the invention to the mammal or other vertebrate. The immune response is preferably protective and preferably involves antibodies. The method may raise a booster response.

The mammal is preferably a human. The subject in which disease is prevented may not be the same as the subject that receives the conjugate of the invention. For instance, a conjugate may be administered to a female (before or during pregnancy) in order to protect offspring (so-called 'maternal immunisation'). Conjugates of the invention may also be used to immunise other mammals *e.g.* cattle, sheep and pigs (especially against *Salmonella* sp.), and other non-mammal vertebrates including fish and poultry.

The invention provides conjugates of the invention for use in therapy (*e.g.* as immunogenic compositions or as vaccines). The invention also provides a conjugate of the invention for use in a method for raising an immune response in a vertebrate, preferably a mammal. The invention also provides the use of a conjugate of the invention in the manufacture of a medicament for raising an immune response in a vertebrate, preferably a mammal. The uses and methods are particularly useful for preventing/treating a variety of diseases, depending on the antigens and nOMVs within the conjugates as above set forth. Preferred conjugates of the invention can confer an antibody titre in a patient that is superior to the criterion for seroprotection for each antigenic component for an acceptable percentage of human subjects. Antigens with an associated antibody titre above which a host is considered to be seroconverted against the antigen are well known, and such titres are published by organisations such as WHO. Preferably more than 80% of a statistically significant sample of subjects is seroconverted, more preferably more than 90%, still more preferably more than 93% and most preferably 96-100%.

Immunogenic compositions of the invention will generally be administered directly to a patient. Direct delivery may be accomplished by parenteral injection (*e.g.* subcutaneously, intraperitoneally, intravenously, intramuscularly, or to the interstitial space of a tissue), or by rectal, oral, vaginal, topical, transdermal, intranasal, ocular, aural, pulmonary or other mucosal administration. Intramuscular administration is preferred *e.g.* to the thigh or the upper arm. Injection may be via a needle (*e.g.* a hypodermic needle), but needle-free injection may alternatively be used. The invention may also be used to elicit systemic and/or mucosal immunity. Dosage treatment can be a single dose schedule or a multiple dose schedule. Multiple doses may be used in a primary immunisation schedule and/or in a booster immunisation schedule. A primary dose schedule may be followed by a booster dose schedule. Suitable timing between priming doses (*e.g.* between 4-16 weeks), and between priming and boosting, can be routinely determined.

Infections affect various areas of the body and so the compositions of the invention may be prepared in various forms. For example, the compositions may be prepared as injectable, either as liquid solutions or suspensions. Solid forms suitable for solution in, or suspension in, liquid vehicles prior to injection can also be prepared. The composition may be prepared for topical administration *e.g.* as an ointment, cream or powder. The composition be prepared for oral administration *e.g.* as a tablet or capsule, or as a syrup (optionally flavoured). The composition may be prepared for pulmonary administration *e.g.* as an inhaler, using a fine powder or a spray. The composition may be prepared as a suppository or pessary. The composition may be prepared for nasal, aural or ocular administration *e.g.* as drops. Compositions suitable for parenteral injection are most preferred. The composition is preferably sterile. It is preferably pyrogen-free. It is preferably buffered *e.g.* at between pH 6 and pH 8, generally around pH 7. Compositions of the invention may be isotonic with respect to humans. Immunogenic compositions comprise an immunologically effective amount of a conjugate of the invention, as well as any other of other specified components, as needed. Dosage treatment may be a single dose schedule or a multiple dose schedule (*e.g.* including booster doses). The composition may be administered in conjunction with other immunoregulatory agents.

Adjuvants which may optionally be used in compositions of the invention include, but are not limited to insoluble metal salts, oil-in-water emulsions *(e.g.* MF59 or AS03, both containing squalene), saponins, non-toxic conjugates of LPS (such as monophosphoryl lipid A or 3-O-deacylated MPL), immunostimulating oligonucleotides, detoxified bacterial ADP-ribosylating toxins, microparticles, liposomes, imidazoquinolones, or mixtures thereof. Other substances that act as immunostimulating agents are disclosed for instance in Watson, Pediatr. Infect. Dis. J. (2000) 19:331-332. The use of an aluminium hydroxide and/or aluminium phosphate adjuvant is particularly preferred. These salts include oxyhydroxides and hydroxyphosphates. The salts can take any suitable form (*e.g.* gel, crystalline, amorphous, etc.).

Conjugates of the invention which include nOMVs from one pathogen and a selected antigen from a second pathogen can be useful as immunogenic compounds for the preparation of multivalent vaccines.

Preferred nOMV-linker-antigen conjugates of the invention are indicated in the following Table 1.

**Table 1: preferred conjugates of the invention**

| ***nOMV*** | ***linker*** | ***Antigen*** |
|---|---|---|
| *Salmonella* Typhimurium | BS3 | Plasmodium falciparum Pfs25 |
| *Salmonella* Typhimurium | BS(PEG)₅ | Plasmodium falciparum Pfs25 |
| *Salmonella* Typhimurium | BS3 | fHbp (*Neisseria meningitidis*) |
| *Salmonella* Typhimurium | BS(PEG)₅ | fHbp (*Neisseria meningitidis*) |
| *Salmonella* Typhimurium | BS3 | Plasmodium falciparum RO6C |
| *Salmonella* Typhimurium | BS(PEG)₅ | Plasmodium falciparum RO6C |
| *Salmonella* Typhimurium | BS3 | Plasmodium falciparum CSP |
| *Salmonella* Typhimurium | BS(PEG)₅ | Plasmodium falciparum CSP |
| *Meningococcal B* | BS3 | fHbp (*Neisseria meningitidis*) |
| *Meningococcal B* | BS(PEG)₅ | fHbp *(Neisseria meningitidis)* |
| *Meningococcal B* | BS3 | NHBA (*Neisseria meningitidis*) |
| *Meningococcal B* | BS(PEG)₅ | NHBA (*Neisseria meningitidis*) |
| *Salmonella* Typhimurium | BS3 | Synthetic or native GAS PS |
| *Salmonella* Typhimurium | BS(PEG)₅ | Synthetic or native GAS PS |
| *Salmonella* Typhimurium | BS3 | Synthetic or native GBS PS |
| Meningococcal B | BS3 | Synthetic or native GAS PS |
| Meningococcal B | BS3 | Hib PS |
| *B. Pertussis* | BS3 | Hib PS |
| *Salmonella* Typhimurium | BS3 | Vi PS |
| *Shigella* | BS3 | ETEC 405 |
| *Shigella* | BS3 | ETEC 3526 |
| *Shigella* | BS3 | ETEC CTF1232 |
| Meningococcal B | SIDEA | Capsular saccharide from MenA |
| Meningococcal B | SIDEA | Capsular saccharide from MenC |

Thus, the present conjugates are particularly useful as immunogenic agents against the pathogens listed in Table 1. In particular, data confirmed that GMMA from MenB conjugated to capsular saccharides from MenA and/or MenC show r/hSBA results comparable or even better that those obtained using CRM197 as carrier protein. The SBAs data in fact confirm the immunogenic response against MenA and MenC as well as against MenB (see in particular Examples 12, 13 and 14).

This means that the present conjugates can be suitable used for the preparation of a multivalent immunogenic composition, or a vaccine, for instance, but not only, against MenB and MenC and/or MenA, as herein described and supported in details.

The invention will be now described by the following experimental part, without posing any limitation to its scope.

### EXPERIMENTAL PART

### EXAMPLE 1: nOMV production

The nOMVs as used in the Examples are GMMA vesicles, prepared from S. Typhimurium or N. *meninigitidis* B strains, as *e.g.* disclosed in Clin Vaccine Immunol. 2016 Apr; 23(4): 304-314Characteristics of said nOMVs were as indicated in the following Table 2.

**Table 2: characteristics of purified nOMVs prepared from S. Typhimurium or N. meninigitidis B strains.**

| | ***S. Typhimurium* 1418 (ΔtoIR)** | ***S. Typhimurium* 2192 (ΔtoIR ΔpagP ΔmsbB)** | ***MenB* (ΔlpxL1, ΔsynX, Δgna33 and ΔfHbp)** |
|---|---|---|---|
| Diameter (nm) by dls | 131.5 | 112.6 | 95.6 |
| nmol Lipid A/mg vesicles by HPLC-SEC/semicarbazide | 172.8 | 243.2 | 68.6 |
| OAg / total protein weight ratio | 0.84 | 0.93 | only LOS present |

### EXAMPLE 2: nOMV conjugation via BS3 linker (Pfs25-S. Typhimurium nOMV conjugation)

Malaria antigen Pfs25 was conjugated to the S. Typhimurium nOMV vesicles following the procedure described below. nOMV, at a protein concentration of 4 mg/mL in 100 mM borate buffer pH 9, were added of BS3 linker (48 mg/mL in the reaction mixture). The mixture was incubated at the controlled temperature of 25°C for 30 minutes. After this time, activated nOMV were purified by ultracentrifuge (110000 rpm, 16 min, 4°C) and immediately added of Pfs25 antigen. In the conjugation step, a 1:1 ratio of nOMV:Pfs25 was used, at a Pfs25 concentration of 2.7 mg/ml in PBS with overnight incubation at room temperature. The conjugate was purified by ultracentrifuge (110000 rpm, 16 min, 4°C) and re-suspended in PBS. Characterization by SDS page/western blot analysis confirmed conjugate formation. Analysis by competitive ELISA showed 19.2% Pfs25 to total protein w/w ratio in the final conjugate. Intermediate activated nOMV were characterised by 62% NH₂ groups derivatised with the linker and 32% active ester groups introduced on NH₂ groups on nOMV. No crosslinking was verified in both the steps of nOMV activation with BS3 and following conjugation, as verified by dls

### EXAMPLE 3: fHbp v2 linked to S. Typhimurium nOMV by BS3 chemistry (recycling fHbp)

fHbp v2 antigen was conjugated to S. Typhimurium nOMV by BS3 chemistry. The reaction was performed by working with fHbp to nOMV-BS3 w/w ratio of 1, fHbp v2 concentration of 0.92 mg/mL in PBS. A further conjugate was produced by recycling unreacted fHbp v2 from the first conjugation batch and re-using it for conjugation with a fresh batch of nOMV-BS3, by using same conjugation condition. SDS PAGE western blot analysis confirmed conjugate formation also with the recycled fHbp v2.

### EXAMPLE 4: CSP and (NANP)₃-SH linked to S. Typhimurium nOMV by SH-malemido chemistry according to the invention

### Example 4.1: CSP derivatization with N-ε-malemidocaproyl-oxysuccinimde ester (EMCS) linker.

CSP at the concentration of 270 µg/mL in PBS was added of EMCS linker (as a 10 mg/mL solution in DMSO) to have a 1:1 molar ratio of linker to Lys residues of the protein. The solution was mixed at RT for 4 hours and then the derivatised protein was purified by PD10 column against MES 10 mM pH 6. The resulting product was characterised by micro BCA (64% recovery).

### Example 4.2: S. Typhimurium nOMV derivatization with SH linker.

nOMV were re-suspended in 100 mM borate buffer pH 8 and added of the activation buffer containing 2.6 mg/mL DTT, 13.16 mg/mL EDTA and 7.04 mg/mL N-acetyl-DL-homocysteine thiolactone linker in 100 mM borate buffer pH 11. nOMV were at 2.3 mg/mL with a molar ratio of linker to NH₂ groups on GMMA equal to 7. nOMV-SH were purified by ultracentrifugation (110000 rpm, 4°C, 1 hour) and recovered in 100 mM MES buffer pH 6. nOMV-SH were characterised by micro BCA (80% protein recovery) and TNBS showing 54% NH₂ groups were activated.

### Example 4.3: S. Typhimurium nOMV derivatization with EMCS linker.

nOMV were resuspended in 100 mM NaPi pH 7.2 and added of EMCS linker (as a 20 mg/mL solution in DMSO) to have a 0.6:1 molar ratio of linker to Lys residues of the protein and with nOMV concentration of 3.94 mg/mL. The reaction was left mixing at RT for 4 hours and nOMV-EMCS were purified by ultracentrifugation (110000 rpm, 4°C, 1 hour). Derivatised nOMV were recovered in 100 mM MES buffer pH 6. nOMV-EMCS were characterised by micro BCA (87% protein recovery) and TNBS and Ellman colorimetric methods for assessing the % of NH₂ groups activated (30%).

### Example 4.4: Conjugation of nOMV-SH with CSP-EMCS.

CSP protein, previously derivatised with EMCS linker, was conjugated to nOMV-SH. Conjugation was performed with 1:1 w/w nOMV to CSP ratio at CSP concentration of 96.7 µg/mL in 100 mM MES pH 6. The reaction was left mixing at RT for 4 hours and the conjugate was purified by ultracentrifuge (110000 rpm, 4°C, 1 hour) and recovered in PBS. Amount of conjugated CSP respect to total protein content was of 0.33 (w/w) as evaluated by HPLC-SEC analysis of the conjugation mixture. SDS PAGE/western blot analysis confirmed conjugate formation.

### Example 4.5: Conjugation of nOMV-EMCS with (NANP)₃-SH.

(NANP)₃-SH protein, having a terminal Cys residue, was added to nOMV-EMCS in MES 100 mM pH 6 at a concentration of 8.7 mg/mL and with a w/w ratio of 1 respect to nOMV. The reaction was left mixing at RT for 4 hours and the conjugate was purified by ultracentrifuge (110000 rpm, 4°C, 1 hour) and recovered in PBS. Amount of conjugated (NANP)₃ respect to total protein content was of 0.31 (w/w) as calculated by Ellman and HPAEC-PAD analysis on the conjugate. SDS PAGE/western blot analysis confirmed conjugate formation.

### EXAMPLE 5: immunogenicity of conjugates of Example 4 in mice

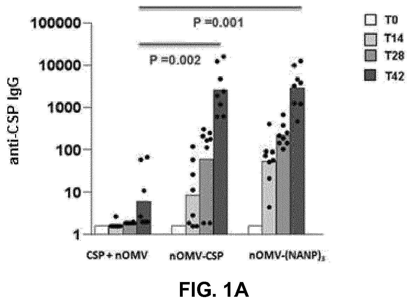

Mice were immunised subcutaneously at days 0 and 28 with nOMV-CSP and nOMV-(NANP)₃ conjugates prepared according to Example 4. Compared to the physical mixture nOMV + CSP, at 2 µg CSP or (NANP)₃ per dose. Anti-CSP IgG titers were measured at days 0, 14, 28 and 42, and results are shown in Figure 1A.

Both nOMV conjugates induced higher anti-CSP specific response than CSP physically mixed with nOMV (Kruskal-Wallis test with Dunn's post hoc analysis, p = 0.002 for nOMV-CSP and p = 0.001 for nOMV-(NANP)₃ respectively at day 42).

Both the conjugates showed the ability to boost the response (day 14-day 42).

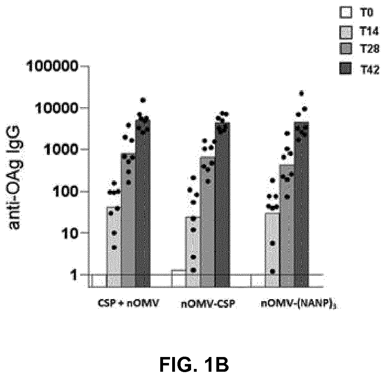

IgG titers against the -OAg were also assessed. The presence of CSP or (NANP)₃ on nOMV surface did not impact on the ability of nOMV to induce specific anti-OAg IgG response (Figure 1B).

### EXAMPLE 6: fHbp v1.1 linked to S. Typhimurium nOMV by SH-malemido or click chemistry

### Example 6.1: S. Typhimurium nOMV derivatization with SH linker.

nOMV were suspended in 100 mM borate buffer pH 8 and added of the activation buffer containing 2.6 mg/mL DTT, 13.16 mg/mL EDTA and 7.04 mg/mL N-acetyl-DL-homocysteine thiolactone linker in 100 mM borate buffer pH 11. nOMV were at 3.0 mg/mL with a molar ratio of linker to NH₂ groups on nOMV equal to 6.63. The reaction was left mixing at RT for 4 hours and nOMV-SH were then purified by dialysis against 50 mM MES 0.5 mM DTT buffer pH 6. nOMV-SH were characterised by Lowry (75% protein recovery) and TNBS showing that 41% of NH₂ groups were activated.

### Example 6.2: fHbp v1.1 derivatization with N-ε-malemidocaproyl-oxysuccinimde ester (EMCS) linker.

fHbp at the concentration of 1.25 mg/mL in PBS was added of EMCS linker (as a 10 mg/mL solution in DMSO) to have a 0.2:1 molar ratio of linker to Lys residues of the protein. The solution was mixed at RT for 4.5 hours and then the derivatised protein was purified by PD10 column against MES 10 mM pH 6. The resulting product was characterised by micro BCA (95% recovery), HPLC-SEC and SDS-PAGE showing no protein aggregation respect to underivatised protein and MALDI-MS indicating an average of three linkers introduced per molecule of protein.

### Example 6.3: Conjugation of nOMV-SH with fHbp-EMCS.

fHbp-EMCS was conjugated to nOMV-SH. Conjugation was performed with 2:1 w/w nOMV to fHbp ratio at fHbp concentration of 2 mg/mL. The reaction was left mixing at RT for 5 hours and the conjugate was purified by Vivaspin 100k against PBS. Conjugate formation was confirmed by SDS page/western blot analysis.

### Example 6.4: S. Typhimurium nOMV derivatisation with alkyne linker.

nOMV were suspended in 100 mM NaPi pH 7.2 and added of click-easy BCN N-hydroxysuccinimidester I (Berry Associates) linker 25 mg/mL in DMSO. nOMV were at 8.9 mg/mL with a molar ratio of linker to NH₂ groups on GMMA equal to 0.6. The reaction was left mixing at RT for 4 hours and nOMV-alkyne were then purified by dialysis against NaPi 100 mM pH 7.2. nOMV-alkyne were characterised by Lowry (72% protein recovery) and TNBS showing that 40% of NH₂ groups were activated.

### Example 6.5: fHbp v1.1 derivatisation with NHS-PEG₄-N₃ linker.

fHbp at the concentration of 1.25 mg/mL in PBS was added of -N₃ linker (as a 25 mg/mL solution in DMSO) to have a 0.2:1 molar ratio of linker to Lys residues of the protein. The solution was mixed at RT for 6 hours and then the derivatised protein was purified by PD10 column against NaPi 10 mM pH 7.2. The resulting product was characterised by micro BCA (87% recovery), HPLC-SEC and SDS-PAGE, showing no protein aggregation respect to the underivatised protein, and MALDI-MS, indicating an average of two linkers introduced per molecule of protein.

### Example 6.6: Conjugation of nOMV-alkyne with fHbp-N₃.

fHbp-N₃ was conjugated to nOMV-alkyne. Conjugation was performed with 2:1 w/w nOMV to fHbp ratio at fHbp concentration of 1.67 mg/mL. The reaction was left mixing at RT for 5 hours and the conjugate was purified by Vivaspin 100k against PBS. Conjugate formation was verified by SDS page/western blot analysis.

### EXAMPLE 7: immunogenicity of conjugates obtained in Example 6 in mice

Mice were immunised subcutaneously at days 0 and 28 with the synthesized conjugates compared to fHbp alone, nOMV alone and fHbp physically mixed with nOMV. 2.5 µg total protein (1.75 µg nOMV and 0.75 µg fHbp, assuming a fHbp to total protein w/w ratio in the conjugates of 0.3) per dose was used. All antigens were formulated with Alhydrogel 0.7 mg/mL Al³⁺ and 10 mM Histidine.

Antibodies induced by the conjugates showed broad protection against different meningococcal strains, with bactericidal activity higher compared with recombinant fHbp alone or physically mixed with nOMV (Table 3). Sera from conjugates also resulted bactericidal against the invasive Malawian clinical isolate D23580 S. Typhimurium strain and not inferior to nOMV alone in terms of anti-OAg IgG response induced (Table 3).

**Table 3. SBA titers against different meningococcal strains of pooled sera collected after immunization with fHbp constructs. Mice were immunized at day 0 and 28 with 2.5 µg total protein (1.75 µg nOMV and 0.75 µg fHbp, assuming a fHbp to total protein w/w ratio in the conjugates of 0.3) formulated with Alhydrogel.**

| | **SBA titers against strains** | | | | | | | |
|---|---|---|---|---|---|---|---|---|
| | **Niga 16/09** | **Bufa 20030020** | **Cam2_09** | **Mali 4/11** | **B H44/76** | | **B M6190** | **D23580** |
| | **fHbp ID 5** | | **fHbp ID 9** | | **fHbp ID 1** | | | **STm** |
| **Sera** | **d42** | **d42** | **d42** | **d42** | **d14** | **d42** | **d42** | **d42** |
| **nOMV-SH-fHbp** | 32000 | 3600 | 2800 | <10 | 1000 | >163840 | >163840 | 66000 |
| **nOMV-click-fHbp** | 40000 | 7000 | 600 | <10 | 2800 | >163840 | 130000 | 17370 |
| **nOMV + fHbp** | 1800 | <10 | <10 | <10 | 40 | >163840 | 10000 | 365732 |
| **fHbp** | 2500 | <10 | <10 | <10 | <10 | >163840 | 2000 | <100 |
| **nOMV** | <10 | <10 | <10 | <10 | <10 | <10 | <10 | 73309 |

### EXAMPLE 8: MenB nOMV conjugated to fHbp-SH v3 protein by BS3 chemistry (no aggregates formation after conjugation)

MenB nOMV, at a protein concentration of 2.8 mg/mL in 100 mM MES buffer pH 6, were added of BS3 linker (50 mg/mL in the reaction mixture). The mixture was incubated at the controlled temperature of 25°C for 30 minutes. After this time, activated nOMV were purified by ultracentrifuge (110000 rpm, 16 min, 4°C) and immediately added of fHbp-SH v3. In the conjugation step, a 1:1 w/w ratio of nOMV to fHbp-SH was used, with nOMV concentration of 1.7 mg/ml in PBS. After overnight mixing at RT, the conjugate was purified by ultracentrifuge (110000 rpm, 16 min, 4°C) and recovered in PBS. Characterization by SDS page/western blot confirmed conjugate formation. nOMV-BS3 and final conjugate were compared to native nOMV by HPLC-SEC/MALS showing no aggregate formation (Table below).

| **Sample** | **Rw (nm)** |
|---|---|
| MenB nOMV | 44.1 |
| MenB nOMV-BS3 | 58.6 |
| MenB nOMV-BS3-fHbp conj | 54.0 |

### EXAMPLE 8a (comparative): dOMV conjugation via BS3 linker

dOMV (from MenB) has been tested as starting material for reaction with BS3 linker, under the following conditions:
- pH: 6.5;
- BS3 concentration: 50 mg/mL;
- dOMV concentration: 1.011 mg/mL;
- 30 min reaction time at 25° C;
- Purification by UC (110Krpm, 16 min, 4°C).

The reaction provides dOMV aggregates and side products as major results. Aggregation/crosslinking has been verified by dls analysis and SEC/MALS.

### EXAMPLE 9: S. Typhimurium nOMV conjugated to GAS-PS

GAS PS was terminally derivatized with ADH linker before conjugation to nOMV. The PS was solubilised in AcONa 20 mM pH 4.5 at 30 mg/mL and added of ADH and NaBH₃CN both with a 1:1 w/w ratio respect to the PS. The solution was mixed at 30°C for 5 days. After this time the product was purified by 2 PD10 column against NaCl 3 M and water, respectively. PS-ADH was characterised by HPAEC-PAD (78% recovery) and TNBS, finding that all the PS chains were activated with ADH linker.

nOMV, at a protein concentration of 4.4 mg/mL in 100 mM MES buffer pH 6, were added of BS3 linker at 50 mg/mL in the reaction mixture. The mixture was incubated at the controlled temperature of 25°C for 30 minutes. After this time, activated nOMV were purified by ultracentrifuge (110000 rpm, 16 min, 4°C) and immediately added of GAS PS-ADH. In the conjugation step, a 1:3 ratio of nOMV to GAS PS-ADH was used, with GAS PS-ADH concentration of 10 mg/ml in PBS. After overnight incubation at RT, the conjugate was purified by ultracentrifuge (110000 rpm, 16 min, 4°C) and recovered in PBS. Characterization by micro BCA, HPAEC-PAD and NTA allowed to estimate an average of 107 PS chains per particle of nOMV.

Same conjugation protocol was applied to conjugate synthetic oligosaccharides of formula (Rha)₄-(CH₂)₃-NH₂ and (Rha)₆-(CH₂)₃-NH₂ to nOMV. Resulting conjugates had an average number of 876 and 640 oligosaccharide chains per nOMV particle.

### EXAMPLE 10: in vivo data of the conjugates of the invention obtained by conjugating a S. Typhimurium nOMV particle to Pfs25 or fHbp v2 antigens by BS3 chemistry.

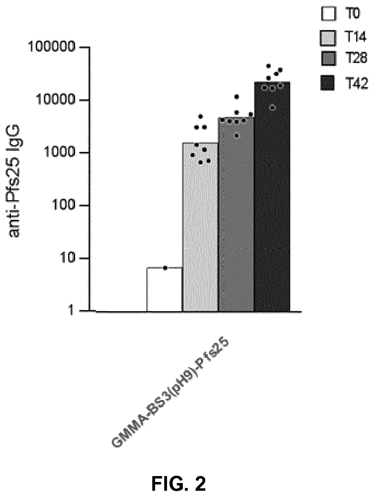

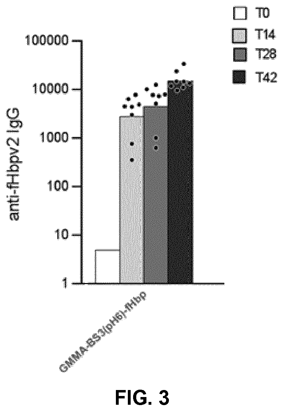

CD1 female mice were immunised subcutaneously at days 0 and 28 with 2.5 µg total protein *S.* Typhimurium nOMV particles conjugated to Pfs25 or fHbp antigens by BS3 chemistry. All the antigens were adsorbed on Alhydrogel (0.7 mg/mL Al³⁺). Anti-Pfs25 (Figure 2) and anti-fHbp v2 (Figure 3) IgG titers were measured at days 0, 14, 27 and 42.

As demonstrated, nOMV-BS3 conjugates were able to induce high anti-antigen specific IgG response.

### EXAMPLE 11: preparation of MenA-GMMA-MenC conjugates via SIDEA likers.

Meningococcal B GMMA overexpressing fHbp, at a protein concentration of 10 mg/mL in 50 mM NaH₂PO₄ buffer pH 7.2, were simultaneously added to MenA and MenC saccharides (avDP 15), previously terminally derivatised with SIDEA linker (Vaccine 1992 10(10):691-698), with a w/w MenA/MenC to nOMV ratio of 10:1, according to procedures know in the art. The mixture was incubated at room temperature overnight. After this time, resulting conjugate was purified by ultracentrifuge (2x, 110000 rpm, 1h, 4°C) and suspended in PBS. Analysis by SDS PAGE western blot confirmed conjugate formation, with both saccharides linked to nOMV. The conjugate was characterised by a w/w MenA to protein ratio and MenC to protein ratio of 0.10 and 0.12 respectively, as determined by HPAEC-PAD and micro BCA analysis, with 19.3% of MenA and 4.3% of MenC free saccharide respectively (by HPAEC-PAD analysis after C4 SPE treatment of the conjugates).

### EXAMPLE 12: preparation of MenA-GMMA conjugates via SIDEA linker

Meningococcal B GMMA overexpressing fHbp, at a protein concentration of 10 mg/mL in 50 mM NaH₂PO₄ buffer pH 7.2, were added to MenA polysaccharide (avDP 15), previously terminally derivatised with SIDEA linker (Vaccine 1992 10(10):691-698), with a w/w MenA to nOMV ratio of 10:1, according to procedures know in the art. The mixture was incubated at room temperature overnight. After this time, resulting conjugate was purified by ultracentrifuge (2x, 110000 rpm, 1h, 4°C) and suspended in PBS. Analysis by SDS PAGE western blot confirmed conjugate formation. The conjugate was characterised by a w/w MenA to protein ratio of 0.054, as determined by HPAEC-PAD and micro BCA analysis, with no free MenA detected by HPAEC-PAD analysis after C4 SPE treatment of the conjugate.

### EXAMPLE 13: preparation of MenC-GMMA conjugates via SIDEA linker

The same procedure used in Example 12 has been followed for the preparation of the titled conjugate, using an antigen from MenC in place of MenA. Conjugate characterization is reported in the Table below.

| **Conjugate** | **OS/GMMA w/w ratio** | **Number OS per GMMA** | **µg/mL OS** | **µg/mL GMMA** | **% free OS** |
|---|---|---|---|---|---|
| GMMA-MenA | 0.054 | 2490 | 102.1 | 1901.6 | not detected |
| GMMA-MenC | 0.1 | 4523 | 195.1 | 1942.6 | not detected |
| Bivalent MenC-GMMA-MenA | 0.103 (MenA) 0.12 (MenC) | 3847 (MenA) 5168 (MenC) | 151.3 (MenA) 176.3 (MenC) | 1468.2 | 4.3% free MenC OS, 19.3% free MenA OS |

### EXAMPLE 14: immunogenicity study in mice

CD1 female mice 5-6 weeks old (8 per group) were immunised intramuscolarly at days 0 and 28 with 1 µg MenA or MenC per dose. GMMA conjugates were compared with MenA-CRM₁₉₇ or MenC-CRM₁₉₇ conjugates, and with GMMA physically mixed to MenA or MenC polysaccharides.

All the antigens were adsorbed on Alhydrogel (0.7 mg/mL Al³⁺). Anti-MenA and MenC IgG titers were measured at days -1, 27 and 42 (Figure 2) and SBA titers of pooled sera from each group at day 42 were measured against a panel of MenA, MenC and MenB strains.

### EXAMPLE 14a: Anti-MenA IgG/SBA

### IgG response

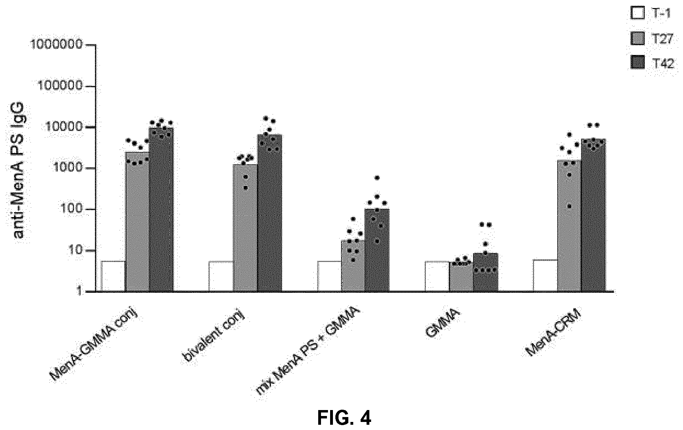

As showed in the Figure 4, the immunogenicity study in mice showed that:
- Higher anti-MenA IgG response is induced by MenA-GMMA conjugate compared to MenA-CRM₁₉₇
- Conjugation of MenA to GMMA enhances the humoral immune response against the polysaccharide.
- The bivalent conjugate induces anti-MenA IgG response comparable to MenA-GMMA conjugate, with no interference due to MenC presence on the same GMMA particles.

### SBA against MenA

| **Antigen** | **MenA-GMMA conj** | **Bivalent conj** | **MenA PS + GMMA** | **GMMA** | **MenA-CRM₁₉₇** |
|---|---|---|---|---|---|
| rSBA MenA strain F8238 | 262144 | 65536 | 1024 | <16 | 8192 |

### EXAMPLE 14b: Anti-MenC IgG/SBA

### Conjugates tested in mice

### IgG response

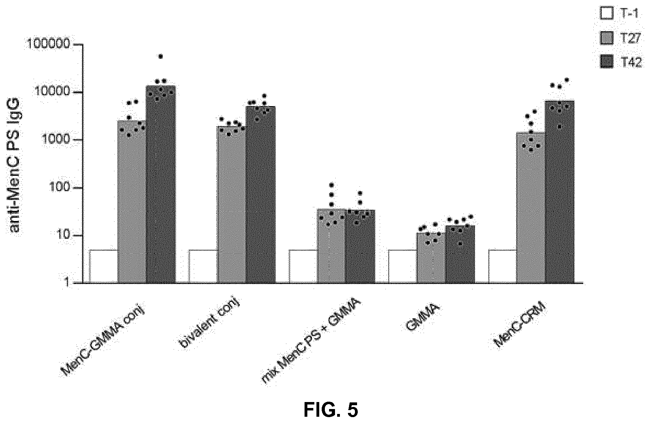

As showed in Figure 5, the immunogenicity study in mice showed that:
- Similar anti-MenC IgG response induced by MenC-GMMA compared to MenC-CRM₁₉₇
- Conjugation of MenC to GMMA enhances the humoral immune response against OS

### SBA against MenC

| **Antigen** | **MenA-GMMA conj** | **Bivalent conj** | **MenA PS + GMMA** | **GMMA** | **MenA-CRM₁₉₇** |
|---|---|---|---|---|---|
| rSBA MenC strain C11 | 524288 | 262144 | 8192 | 4096 | 32768 |

### EXAMPLE 14c: Anti-MenB SBA

### SBA against MenB for GMMA conjugated to MenA and/or MenC

| **Antigen** | **MenB strain UK 320** | **MenB strain UK 104** |
|---|---|---|
| GMMA-MenA conjugate | 16384 | 32768 |
| GMMA-MenC conjugate | 32768 | 16384 |
| Bivalent conjugate | 1024 | 1024 |
| GMMA | 131072 | 65536 |
| MenA-CRM | <16 | <16 |
| MenC-CRM | <16 | <16 |

The above data show that the conjugation of one or more antigens to the same GMMA according to the invention still provide very good immunogenic profile of the antigens or the GMMA. This means that the present conjugates do not show interference of the antigens, confirming the potential use of the conjugates for the preparation of polyvalent immunogenic composition or vaccine against a variety of pathogens.

### SEQUENCE LISTING

**>SEQ ID NO: 1 [fHbp v2]** 
VAADIGAGLADALTAPLDHKDKSLQSLTLDQSVRKNEKLKLAAQGAEKTYGNGDSLNTGKLKN
DKVSRFDFIRQIEVDGQLITLESGEFQIYKQDHSAVVALQIEKINNPDKIDSLINQRSFRVSGLGG
EHTAFNQLPDGKAEYHGKAFSSDDAGGKLTYTIDFAAKQGHGKIEHLKTPEQNVELAAAELKA
DEKSHAVILGDTRYGSEEKGTYHLALFGDRAQEIAGSATVKIGEKVHEIGIAGKQ
**>SEQ ID NO: 2 [NHBA]** 
MFKRSVIAMACIFALSACGGGGGGSPDVKSADTLSKPAAPVVSEKETEAKEDAPQAGSQGQG
APSAQGSQDMAAVSEENTGNGGAVTADNPKNEDEVAQNDMPQNAAGTDSSTPNHTPDPNM
LAGNMENQATDAGESSQPANQPDMANAADGMQGDDPSAGGQNAGNTAAQGANQAGNNQA
AGSSDPIPASNPAPANGGSNFGRVDLANGVLIDGPSQNITLTHCKGDSCSGNNFLDEEVQLKS
EFEKLSDADKISNYKKDGKNDKFVGLVADSVQMKGINQYIIFYKPKPTSFARFRRSARSRRSLP
AEMPLIPVNQADTLIVDGEAVSLTGHSGNIFAPEGNYRYLTYGAEKLPGGSYALRVQGEPAKGE
MLAGAAVYNGEVLHFHTENGRPYPTRGRFAAKVDFGSKSVDGIIDSGDDLHMGTQKFKAAIDG
NGFKGTWTENGSGDVSGKFYGPAGEEVAGKYSYRPTDAEKGGFGVFAGKKEQD
**>SEQ ID NO: 3 [NadA]** 
MKHFPSKVLTTAILATFCSGALAATSDDDVKKAATVAIVAAYNNGQEINGFKAGETIYDIGEDGTI
TQKDATAADVEADDFKGLGLKKVVTNLTKTVNENKQNVDAKVKAAESEIEKLTTKLADTDAALA
DTDAALDETTNALNKLGENITTFAEETKTNIVKIDEKLEAVADTVDKHAEAFNDIADSLDETNTKA
DEAVKTANEAKQTAEETKQNVDAKVKAAETAAGKAEAAAGTANTAADKAEAVAAKVTDIKADIA
TNKADIAKNSARIDSLDKNVANLRKETRQGLAEQAALSGLFQPYNVGRFNVTAAVGGYKSESA
VAIGTGFRFTENFAAKAGVAVGTSSGSSAAYHVGVNYEW
**>SEQ ID NO: 4 [NspA]** 
MKKALATLIALALPAAALAEGASGFYVQADAAHAKASSSLGSAKGFSPRISAGYRINDLRFAVDY
TRYKNYKAPSTDFKLYSIGASAIYDFDTQSPVKPYLGARLSLNRASVDLGGSDSFSQTSIGLGVL
TGVSYAVTPNVDLDAGYRYNYIGKVNTVKNVRSGELSAGVRVKF
**>SEQ ID NO: 5 [NhhA]** 
MNKIYRIIWNSALNAWVVVSELTRNHTKRASATVKTAVLATLLFATVQASANNEEQEEDLYLDP
VQRTVAVLIVNSDKEGTGEKEKVEENSDWAVYFNEKGVLTAREITLKAGDNLKIKQNGTNFTYS
LKKDLTDLTSVGTEKLSFSANGNKVNITSDTKGLNFAKETAGTNGDTTVHLNGIGSTLTDTLLNT
GATTNVTNDNVTDDEKKRAASVKDVLNAGWNIKGVKPGTTASDNVDFVRTYDTVEFLSADTKT
TTVNVESKDNGKKTEVKIGAKTSVIKEKDGKLVTGKDKGENGSSTDEGEGLVTAKEVIDAVNKA
GWRMKTTTANGQTGQADKFETVTSGTNVTFASGKGTTATVSKDDQGNITVMYDVNVGDALNV
NQLQNSGWNLDSKAVAGSSGKVISGNVSPSKGKMDETVNINAGNNIEITRNGKNIDIATSMTPQ
FSSVSLGAGADAPTLSVDGDALNVGSKKDNKPVRITNVAPGVKEGDVTNVAQLKGVAQNLNN
RIDNVDGNARAGIAQAIATAGLVQAYLPGKSMMAIGGGTYRGEAGYAIGYSSISDGGNWIIKGT
ASGNSRGHFGASASVGYQW
**>SEQ ID NO: 6 [App]** 
MKTTDKRTTETHRKAPKTGRIRFSPAYLAICLSFGILPQAWAGHTYFGINYQYYRDFAENKGKF
AVGAKDIEVYNKKGELVGKSMTKAPMIDFSVVSRNGVAALVGDQYIVSVAHNGGYNNVDFGAE
GRNPDQHRFTYKIVKRNNYKAGTKGHPYGGDYHMPRLHKFVTDAEPVEMTSYMDGRKYIDQN
NYPDRVRIGAGRQYWRSDEDEPNNRESSYHIASAYSWLVGGNTFAQNGSGGGTVNLGSEKIK
HSPYGFLPTGGSFGDSGSPMFIYDAQKQKWLINGVLQTGNPYIGKSNGFQLVRKDWFYDEIFA
GDTHSVFYEPRQNGKYSFNDDNNGTGKINAKHEHNSLPNRLKTRTVQLFNVSLSETAREPVYH
AAGGVNSYRPRLNNGENISFIDEGKGELILTSNINQGAGGLYFQGDFTVSPENNETWQGAGVHI
SEDSTVTWKVNGVANDRLSKIGKGTLHVQAKGENQGSISVGDGTVILDQQADDKGKKQAFSEI
GLVSGRGTVQLNADNQFNPDKLYFGFRGGRLDLNGHSLSFHRIQNTDEGAMIVNHNQDKEST
VTITGNKDIATTGNNNSLDSKKEIAYNGWFGEKDTTKTNGRLNLVYQPAAEDRTLLLSGGTNLN
GNITQTNGKLFFSGRPTPHAYNHLNDHWSQKEGIPRGEIVWDNDWINRTFKAENFQIKGGQAV
VSRNVAKVKGDWHLSNHAQAVFGVAPHQSHTICTRSDWTGLTNCVEKTITDDKVIASLTKTDIS
 
GNVDLADHAHLNLTGLATLNGNLSANGDTRYTVSHNATQNGNLSLVGNAQATFNQATLNGNT
SASGNASFNLSDHAVQNGSLTLSGNAKANVSHSALNGNVSLADKAVFHFESSRFTGQISGGKD
TALHLKDSEWTLPSGTELGNLNLDNATITLNSAYRHDAAGAQTGSATDAPRRRSRRSRRSLLS
VTPPTSVESRFNTLTVNGKLNGQGTFRFMSELFGYRSDKLKLAESSEGTYTLAVNNTGNEPAS
LEQLTVVEGKDNKPLSENLNFTLQNEHVDAGAWRYQLIRKDGEFRLHNPVKEQELSDKLGKAE
AKKQAEKDNAQSLDALIAAGRDAVEKTESVAEPARQAGGENVGIMQAEEEKKRVQADKDTALA
KQREAETRPATTAFPRARRARRDLPQLQPQPQPQPQRDLISRYANSGLSEFSATLNSVFAVQD
ELDRVFAEDRRNAVWTSGIRDTKHYRSQDFRAYRQQTDLRQIGMQKNLGSGRVGILFSHNRT
ENTFDDGIGNSARLAHGAVFGQYGIDRFYIGISAGAGFSSGSLSDGIGGKIRRRVLHYGIQARYR
AGFGGFGIEPHIGATRYFVQKADYRYENVNIATPGLAFNRYRAGIKADYSFKPAQHISITPYLSLS
YTDAASGKVRTRVNTAVLAQDFGKTRSAEWGVNAEIKGFTLSLHAAAAKGPQLEAQHSAGIKL
GYRW
**>SEQ ID NO: 7 [NadA fragment]** 
ATNDDDVKKAATVAIAAAYNNGQEINGFKAGETIYDIDEDGTITKKDATAADVEADDFKGLGLKK
VVTNLTKTVNENKQNVDAKVKAAESEIEKLTTKLADTDAALADTDAALDATTNALNKLGENITTF
AEETKTNIVKIDEKLEAVADTVDKHAEAFNDIADSLDETNTKADEAVKTANEAKQTAEETKQNVD
AKVKAAETAAGKAEAAAGTANTAADKAEAVAAKVTDIKADIATNKDNIAKKANSADVYTREESD
SKFVRIDGLNATTEKLDTRLASAEKSIADHDTRLNGLDKTVSDLRKETRQGLAEQAALSGLFQP
YNVG
**>SEQ ID NO: 8 [fHbp v1]** 
VAADIGAGL ADALTAPLDH KDKGLQSLTL DQSVRKNEKL KLAAQGAEKT YGNGDSLNTG
KLKNDKVSRF DFIRQIEVDG QLITLESGEF QVYKQSHSAL TAFQTEQIQD SEHSGKMVAK
RQFRIGDIAG EHTSFDKLPE GGRATYRGTA FGSDDAGGKL TYTIDFAAKQ GNGKIEHLKS
PELNVDLAAA DIKPDGKRHA VISGSVLYNQ AEKGSYSLGI FGGKAQEVAG SAEVKTVNGI
RHIGLAAKQ
**>SEQ ID NO: 9 [fHbp v3]** 
VAADIGTGLADALTAPLDHKDKGLKSLTLEDSIPQNGTLTLSAQGAEKTFKAGDKDNSLNTGKL
KNDKISRFDFVQKIEVDGQTITLASGEFQIYKQNHSAVVALQIEKINNPDKTDSLINQRSFLVSGL
GGEHTAFNQLPGGKAEYHGKAFSSDDPNGRLHYSIDFTKKQGYGRIEHLKTLEQNVELAAAEL
KADEKSHAVILGDTRYGSEEKGTYHLALFGDRAQEIAGSATVKIGEKVHEIGIAGKQ
**>SEQ ID NO: 10 [Pfs25]** 
KVTVDTVCKR GFLIQMSGHL ECKCENDLVL VNEETCEEKV LKCDEKTVNK PCGDFSKCIK
IDGNPVSYAC KCNLGYDMVN NVCIPNECKQ VTCGNGKCIL DTSNPVKTGV CSCNIGKVPN
VQDQNKCSKD GETKCSLKCL KEQETCKAVD GIYKCDCKDG FIIDQESSIC T
**>SEQ ID NO: 11 [RO6C]** 
AERSTSENRNKRIGGPKLRGNVTSNIKFPSDNKGKIIRGSNDKLNKNSEDVLEQSEKSLVSENV
PSGLDIDDIPKESIFIQEDQEGQTHSELNPETSEHSKDLNNNGSKNESSDIISENNKSNKVQNHF
ESLSDLELLENSSQDNLDKDTISTEPFPNQKHKDLQQDLNDEPLEPFPTQIHKDYKEKNLINEED
SEPFPRQKHKKVDNHNEEKNVFHENGSANGNQGSLKLKSFDEHLKDEKIENEPLVHENLSIPN
DPIEQILNQPEQETNIQEQLYNEKQNVEEKQNSQIPSLDLKEPTNEDILPNHNPLENIKQSESEIN
HVQDHALPKENIIDKLDNQKEHIDQSQHNINVLQENNINNHQLEPQEKPNIESFEPKNIDSEIILPE
NVETEEIIDDVPSPKHSNHETFEEETSESEHEEAVSEKNAHETVEHEETVSQESNPEKADNDG
NVSQNSNNELNENEFVESEKSEHEARSKPKYEKKVIHGCNFSSNVSSKHTFTDSLDISLVDDSA
HISCNVHLSEPKYNHLVGLNCPGDIIPDCFFQVYQPESEELEPSNIVYLDSQINIGDIEYYEDAEG
DDKIKLFGIVGSIPKTTSFTCICKKDKKSAYMTVTIDSARSHHHHHH
**>SEQ ID NO: 12 [CSP]** 
MLFQEYQCYGSSSNTRVLNELNYDNAGTNLYNELEMNYYGKQENWYSLKKNSRSLGENDDG
NNNNGDNGREGKDEDKRDGNNEDNEKLRKPKHKKLKQPGDGNPDPNANPNVDPNANPNVD
PNANPNVDPNANPNANPNANPNANPNANPNANPNANPNANPNANPNANPNANPNANPNANP
NANPNANPNVDPNANPNANPNANPNANPNANPNANPNANPNANPNANPNANPNANPNANPN
 
ANPNANPNANPNANPNANPNANPNANPNKNNQGNGQGHNMPNDPNRNVDENANANNAVKN
NNNEEPSDKHIEKYLKKIQNSLSTEWSPCSVTCGNGIQVRIKPGSANKPKDELDYENDIEKKICK
MEKCSSVFNVVNSSIGLILEHHHHHH
**>SEQ ID NO: 13 [(NANP)3]**
   **NANPNANPNANP**
**>SEQ ID NO: 14 [CTF1232]** 
QDQRYISIRNTDTIWLPGNICAYQFRLDNGGNDEGFGPLTITLQLKDKYGQTLVTRKMETEAFG
DSNATRTTDAFLETECVENVATTEIIKATEESNGHRVSLPLSVFDPQDYHPLLITVSGKNVNLEH
HHHHH
**>SEQ ID NO: 15 [3526, SsIE]** 
DTPSVDSGSGTLPEVKPDPTPTPEPTPEPTPDPEPTPDPTPDPEPTPEPEPEPVPTKTGYLTLG
GSQRVTGATCNGESSDGFTFTPGNTVSCVVGSTTIATFNTQSEAARSLRAVDKVSFSLEDAQE
LANSENKKTNAISLVTSSDSCPADAEQLCLTFSSVVDRARFEKLYKQIDLATDNFSKLVNEEVEN
NAATDKAPSTHTSTVVPVTTEGTKPDLNASFVSANAEQFYQYQPTEIILSEGQLVDSLGNGVAG
VDYYTNSGRGVTDENGKFSFSWGETISFGIDTFELGSVRGNKSTIALTELGDEVRGANIDQLIH
RYSTTGQNNTRVVPDDVRKVFAEYPNVINEIINLSLSNGATLDEGDQNVVLPNEFIEQFKTGQA
KEIDTAICAKTDGCNEARWFSLTTRNVNDGQIQGVINKLWGVDTNYQSVSKFHVFHDSTNFYG
STGNARGQAVVNISNSAFPILMARNDKNYWLAFGEKRAWDKNELAYITEAPSIVQPENVT
RDTATFNLPFISLGQVGEGKLMVIGNPHYNSILRCPNGYSWGGGVNSKGECTLSGDSDDM
KHFMQNVLRYLSNDIWQPNTKSIMTVGTNLENVYFKKAGQVLGNSAPFAFHEDFTGITVK
QLTSYGDLNPEEIPLLILNGFEYVTQWSGDPYAVPLRADTSKPKLTQQDVTDLIAYLNKG
GSVLIMENVMSNLKEESASSFVRLLDAAGLSMALNKSVVNNDPQGYPDRVRQRRATGIWV
YERYPAADGAQPPYTIDPNTGEVTWKYQQDNKPDDKPKLEVASWQEEVEGKQVTRYAFID
EAEYTTEESLEAAKAKIFEKFPGLQECKDSTYHYEINCLERRPGTDVPVTGGMYVPRYTQ
LNLDADTAKAMVQAADLGTNIQRLYQHELYFRTKGSKGERLNSVDLERLYQNMSVWLWND
TKYRYEEGKEDELGFKTFTEFLNCYANDAYAGGTKCSADLKKSLVDNNMIYGDGSSKAGM
MNPSYPLNYMEKPLTRLMLGRSWWDLNIKVDVEKYPGSVSAKGESVTENISLYSNPTKWF
AGNMQSTGLWAPAQQDVTIKSSASVPVTVTVALADDLTGREKHEVALNRPPRVTKTYTLE
ANGEVTFKVPYGGLIYIKGDSKDDVSANFTFTGVVKAPFYKDGEWKNDLDSPAPLGELES
ASFVYTTPKKNLEASNFTGGVAEFAKDLDTFASSMNDFYGRNDEDGKHRMFTYKNLTGHK
HRFTNDVQISIGDAHSGYPVMNSSFSTNSTTLPTTPLNDWLIWHEVGHNAAETPLNVPGA
TEVANNVLALYMQDRYLGKMNRVADDITVAPEYLDESNGQAWARGGAGDRLLMYAQLKEW
AEENFDIKQWYPDGELPKFYSDRKGMKGWNLFQLMHRKARGDDVGNSTFGGKNYCAESNG
NAADTLMLCASWVAQADLSEFFKKWNPGASAYQLPGATEMSFQGGVSSSAYSTLASLKLP
KPEKGPETINKVTEHKMSAE
**>SEQ ID NO: 16 [405, FdeC]**

VADGQQAYTLTLTAVDSEGNPVTGEASRLRLVPQDTNGVTVGAISEIKPGVYSATVSSTR
AGNVVVRAFSEQYQLGTLQQTLKFVAGPLDAAHSSITLNPDKPVVGGTVTAIWTAKDAND
NPVTGLNPDAPSLSGAAAAGSTASGWTDNGDGTWTAQISLGTTAGELDVMPKLNGQDAAA
NAAKVTVVADALSSNQSKVSVAEDHVKAGESTTVTLVAKDAHGNAISGLSLSASLTGTAS
EGATVSSWTEKGDGSYVATLTTGGKTGELRVMPLFNGQPAATEAAQLTVIAGEMSSANST
LVADNKTPTVKTTTELTFTMKDAYGNPVTGLKPDAPVFSGAASTGSERPSAGNWTEKGNG
VYVSTLTLGSAAGQLSVMPRVNGQNAVAQPLVLNVAGDASKAEIRDMTVKVNNQ

## Claims

1. An immunogenic conjugate comprising a native Outer Membrane Vesicle (nOMV) having at least a surface protein moiety connected to at least an antigen by a divalent linker wherein said divalent linker is a homobifunctional linker.

2. The immunogenic conjugate according to claim 1, comprising a nOMV having at least a surface protein moiety connected to a first antigen by a divalent linker, wherein said first antigen is further connected to a second different antigen.

3. The immunogenic conjugate according to claim 1, comprising a nOMV having at least a surface protein moiety connected to a first antigen by a divalent linker, and at least another surface protein moiety connected to a second different antigen by a divalent linker.

4. The immunogenic conjugate according to the preceding claims, wherein said divalent linker has the general formula (I):
X-L-X' **(I)**
wherein:
X and X' are the same, and are a functional group able to selectively react with the nOMV protein residue on one hand and with the antigen on the other hand;
-L- is a divalent linear or branched C₁-C₁₅ alkyl or alkenyl group optionally substituted, and optionally interrupted by one or more heteroatom selected from: oxygen (-O-), sulfur (-S-), nitrogen (-NH- or optionally substituted -N- group).

5. The immunogenic conjugate according to any one of the preceding claims, wherein said divalent linker is selected from at least one of: disuccinimidyl glutarate (DSG), disuccinimidyl suberate (DSS), N-hydroxysuccinimide, N oxysuccinimide and adipic acid N-hydroxysuccinimide diester (SIDEA) and Bis(sulfosuccinimidyl) suberate (BS3), ethylene glycol bis[succinimidylsuccinate], bis(sulfosuccinimidyl)tri(ethylene glycol) (BS(PEG)3), bis(sulfosuccinimidyl)tetra(ethylene glycol) (BS(PEG)4), bis(sulfosuccinimidyl)penta(ethylene glycol) (BS(PEG)5) and bis(sulfosuccinimidyl)exa(ethylene glycol) (BS(PEG)6), adipic acid dihydrazide (ADH), .

6. The immunogenic conjugate according to the preceding claims, wherein said nOMV is obtained by a detergent free process, being released into the fermentation broth and purified using a centrifugation and subsequent filtration; or being released into the fermentation broth and purified using two consecutive Tangential Flow Filtration (TFF) steps.

7. The immunogenic conjugate according to claims 1-6, wherein said nOMV is a GMMA vesicle.

8. A process for preparing the immunogenic conjugate according to claims 1-7, comprising the steps of:
i) reacting at least a nOMV surface protein residue with the first terminal portion of a divalent linker to obtain a nOMV-linker intermediate; and
ii) reacting said nOMV-linker intermediate with at least one antigen via the second terminal portion of the divalent linker, thus obtaining the nOMV-linker-antigen conjugate.

9. The nOMV-linker intermediate obtained (or obtainable) by the step i) of claim 8.

10. Use of the nOMV-linker intermediate of claim 9 for the preparation of nOMV-linker-antigen according to claims 1-7.

11. An immunogenic composition comprising a conjugate according to any one of claims 1-7 and at least one pharmaceutically acceptable carrier or excipient.

12. The immunogenic conjugate or composition according to any one of claims 1-7 or 11, for use as medicament.

13. The immunogenic conjugate or composition for use according to claim 12 to induce an immune response in a vertebrate.

14. A vaccine comprising the immunogenic conjugates defined in any one of claims 1-7 or the immunogenic composition defined in claim 11.

## Patentansprüche

1. Immunogenes Konjugat, umfassend ein natives Außenmembranvesikel (native Outer Membrane Vesicle; nOMV) mit wenigstens einer Oberflächenproteineinheit, die durch einen divalenten Linker mit wenigstens einem Antigen verbunden ist, wobei der divalente Linker ein homobifunktioneller Linker ist.

2. Immunogenes Konjugat gemäß Anspruch 1, umfassend ein nOMV mit wenigstens einer Oberflächenproteineinheit, die durch einen divalenten Linker mit einem ersten Antigen verbunden ist, wobei das erste Antigen weiterhin mit einem zweiten, unterschiedlichen Antigen verbunden ist.

3. Immunogenes Konjugat gemäß Anspruch 1, umfassend ein nOMV mit wenigstens einer Oberflächenproteineinheit, die durch einen divalenten Linker mit einem ersten Antigen verbunden ist, und wenigstens einer anderen Oberflächenproteineinheit, die durch einen divalenten Linker mit einem zweiten, unterschiedlichen Antigen verbunden ist.

4. Immunogenes Konjugat gemäß den vorangegangenen Ansprüchen, wobei der divalente Linker die allgemeine Formel (I) hat:
X-L-X' (I)
worin:
X und X' gleich sind und eine funktionelle Gruppe sind, die zum selektiven Reagieren mit dem nOMV-Proteinrest einerseits und mit dem Antigen andererseits in der Lage sind;
-L- eine divalente lineare oder verzweigte C₁-C₁₅-Alkyl- oder Alkenylgruppe ist, optional substituiert und optional unterbrochen durch ein oder mehrere Heteroatom(e), ausgewählt aus: Sauerstoff (-0-), Schwefel (-S-), Stickstoff (-NH- oder optional substituierte -N--Gruppe).

5. Immunogenes Konjugat gemäß irgendeinem der vorangegangenen Ansprüche, wobei der divalente Linker aus wenigstens einem der folgenden ausgewählt ist:
Disuccinimidylglutarat (DSG), Disuccinimidylsuberat (DSS), N-Hydroxysuccinimid, N-Oxysuccinimid und Adipinsäure N-hydroxysuccinimiddiester (SIDEA) und Bis(sulfosuccinimidyl)suberat (BS3), Ethylenglycol bis[succinimidylsuccinat],
Bis(sulfosuccinimidyl)tri(ethylenglycol) (BS(PEG)3), Bis(sulfosuccinimidyl)tetra(ethylenglycol) (BS(PEG)4), Bis(sulfosuccinimidyl)penta(ethylenglycol) (BS(PEG)5) und Bis(sulfosuccinimidyl)exa(ethylenglycol) (BS(PEG)6), Adipinsäuredihydrazid (ADH).

6. Immunogenes Konjugat gemäß den vorangegangenen Ansprüchen, wobei das nOMV durch ein detergensfreies Verfahren erhalten wird, in die Fermentationsbrühe freigesetzt und unter Verwendung einer Zentrifugation und anschließender Filtration aufgereinigt wird; oder in die Fermentationsbrühe freigesetzt und unter Verwendung zweier aufeinanderfolgender Tangentialflussfiltrations (TFF)-Schritte aufgereinigt wird.

7. Immunogenes Konjugat gemäß den Ansprüchen 1-6, wobei das nOMV ein GMMA-Vesikel ist.

8. Verfahren zum Herstellen des immunogenen Konjugats gemäß den Ansprüchen 1-7, welches die folgenden Schritte umfasst:
i) Reagieren wenigstens eines nOMV-Oberflächenproteinrests mit dem ersten terminalen Abschnitt eines divalenten Linkers, um ein nOMV-Linker-Intermediat zu erhalten; und
ii) Reagieren des nOMV-Linker-Intermediats mit wenigstens einem Antigen über den zweiten terminalen Abschnitt des divalenten Linkers und dadurch Erhalten des nOMV-Linker-Antigen-Konjugats.

9. nOMV-Linker-Intermediat, erhalten (oder erhältlich) durch den Schritt i) aus Anspruch 8.

10. Verwendung des nOMV-Linker-Intermediats nach Anspruch 9 zur Herstellung von nOMV-Linker-Antigen gemäß den Ansprüchen 1-7.

11. Immunogene Zusammensetzung, umfassend ein Konjugat gemäß irgendeinem der Ansprüche 1-7 und wenigstens einen pharmazeutisch akzeptablen Träger oder Hilfsstoff.

12. Immunogenes Konjugat oder Zusammensetzung gemäß irgendeinem der Ansprüche 1-7 oder 11, zur Verwendung als Medikament

13. Immunogenes Konjugat oder Zusammensetzung zur Verwendung gemäß Anspruch 12, um eine Immunantwort in einem Wirbeltier zu induzieren.

14. Impfstoff, umfassend die in irgendeinem der Ansprüche 1-7 definierten immunogenen Konjugate oder die in Anspruch 11 definierte immunogene Zusammensetzung.

## Revendications

1. Conjugué immunogène comprenant une vésicule de membrane externe native (VMEn) présentant au moins une fraction de protéine de surface reliée à au moins un antigène par un lieur divalent dans lequel ledit lieur divalent est un lieur homobifonctionnel.

2. Conjugué immunogène selon la revendication 1, comprenant une VMEn présentant au moins une fraction de protéine de surface reliée à un premier antigène par un lieur divalent, dans lequel ledit premier antigène est relié en outre à un second antigène différent.

3. Conjugué immunogène selon la revendication 1, comprenant une VMEn présentant au moins une fraction de protéine de surface reliée à un premier antigène par un lieur divalent, et au moins une autre fraction de protéine de surface reliée à un second antigène différent par un lieur divalent.

4. Conjugué immunogène selon les revendications précédentes, dans lequel ledit lieur divalent présente la formule générale (I):
X-L-X' (I)
dans lequel :
X et X' sont identiques, et représentent un groupe fonctionnel capable de réagir sélectivement, d'une part, avec le résidu de protéine de la VMEn et d'autre part, avec l'antigène ;
-L- représente un groupe alcényle ou alkyle en C₁ à C₁₅ linéaire ou ramifié divalent substitué facultativement, et interrompu facultativement par un ou plusieurs hétéroatomes choisis parmi : oxygène (-O-), soufre (-S-), azote (-NH- ou substitué facultativement par le groupe -N-).

5. Conjugué immunogène selon l'une quelconque des revendications précédentes, dans lequel ledit lieur divalent est choisi parmi au moins l'un des éléments suivants : glutarate de disuccinimidyle (DSG), subérate de disuccinimidyle (DSS), N-hydroxysuccinimide, N-oxysuccinimide et diester de N-hydroxysuccinimide d'acide adipique (SIDEA) et subérate de bis(sulfosuccinimidyle) (BS3), bis[succinimidylsuccinate] d'éthylène glycol, bis(sulfosuccinimidyl)tri(éthylène glycol) (BS(PEG)3), bis(sulfosuccinimidyl)tétra(éthylène glycol) (BS(PEG)4), bis(sulfosuccinimidyl)penta(éthylène glycol) (BS(PEG)5) et bis(sulfosuccinimidyl)exa(éthylène glycol) (BS(PEG)6), dihydrazide d'acide adipique (ADH).

6. Conjugué immunogène selon les revendications précédentes, dans lequel ladite VMEn est obtenue par un procédé sans détergent, étant libéré à l'intérieur du bouillon de fermentation et purifié en utilisant une centrifugation et une filtration ultérieure ; ou étant libéré à l'intérieur du bouillon de fermentation et purifié en utilisant deux étapes consécutives de filtration à flux tangentiel (FFT).

7. Conjugué immunogène selon les revendications 1 à 6, dans lequel ladite VMEn est une vésicule GMMA.

8. Procédé de préparation du conjugué immunogène selon les revendications 1-7, comprenant les étapes consistant à :
i) faire réagir au moins un résidu de protéine de surface de la VMEn avec la première partie terminale d'un lieur divalent pour obtenir un intermédiaire VMEn-lieur ; et
ii) faire réagir ledit intermédiaire VMEn-lieur avec au moins un antigène par le biais de la seconde partie terminale du lieur divalent, permettant ainsi d'obtenir le conjugué VMEn-lieur-antigène.

9. Intermédiaire VMEn-lieur obtenu (ou pouvant être obtenu) par l'étape i) selon la revendication 8.

10. Utilisation de l'intermédiaire VMEn-lieur selon la revendication 9 destinée à la préparation de VMEn-lieur-antigène selon les revendications 1-7.

11. Composition immunogène comprenant un conjugué selon l'une quelconque des revendications 1-7 et au moins un vecteur ou un excipient pharmaceutiquement acceptable.

12. Conjugué ou composition immunogène selon l'une quelconque des revendications 1-7 ou 11, destiné(e) à être utilisé(e) en tant que médicament.

13. Conjugué ou composition immunogène destiné(e) à être utilisé(e) selon la revendication 12 pour induire une réponse immunitaire chez un vertébré.

14. Vaccin comprenant les conjugués immunogènes définis selon l'une quelconque des revendications 1-7 ou composition immunogène définie selon la revendication 11.
